# EUROPEAN PATENT SPECIFICATION

(11) **EP 2 129 406 B1**
(45) Date of publication and mention of the grant of the patent: **12.08.2015**
(21) Application number: 08729687.7
(22) Date of filing: 12.02.2008
(51) Int. Cl.: A61L 15/32, A61L 15/40, A61L 27/24, A61L 27/36, A61L 31/00, A61L 31/04

(54) **COLLAGEN PRODUCTS AND METHODS FOR PRODUCING COLLAGEN PRODUCTS**
COLLAGENPRODUKTE UND VERFAHREN ZUR HERSTELLUNG VON COLLAGENPRODUKTEN
PRODUITS AU COLLAGÈNE ET PROCÉDÉS DE FORMATION DESDITS PRODUITS

(30) Priority: 12.02.2007 US 673972; 07.09.2007 US 970721 P
(43) Date of publication of application: 09.12.2009
(73) Proprietor: Warsaw Orthopedic, Inc., Warsaw, IN 46581 (US)
(72) Inventor: LAURITZEN, Nels, J., Piscataway, NJ 08854 (US); SHIMP, Lawrence, A., Morganville, NJ 07751 (US); MITCHELL, Brent, New Jersey 08873 (US)
(74) Representative: O'Connell, Maura
(86) International application number: PCT/US2008/053763
(87) International publication number: WO 2008/100967

(56) References cited:
- EP-A2- 0 781 564
- WO-A-00/35375
- WO-A-85/05274
- WO-A-96/31157
- BURRES, STEVEN: "Preserved Particulate Fascia Lata for Injection: A New Alternative" DERMATOL SURG, vol. 25, October 1999 (1999-10), pages 790-794, XP002535215 cited in the application

## Description

### FIELD OF THE INTENTION

The invention relates generally to a method for preparing human-derived collagen fibers and/or threads and collagen implants using the collagen fibers and/or threads.

### BACKGROUND

Collagen is used as an implant material to replace or augment hard or soft connective tissue, such as skin, tendons, cartilage, and bone. Some implants are formed as solid, flexible, or deformable collagen masses cross-linked with chemical agents, radiation, or other means to improve mechanical properties, decrease the chance of an immunogenic response, and/or to manage the resorption rate.

Collagen-based medical implants for use in humans generally have been of a non-human origin, i.e., xenogenic. A problem with the use of xenogenic tissue as a starting material when generating medical implants is that the tissue may be contaminated with viruses or prions. For example, products using bovine sourced tissue have the potential for transmitting BSE (Bovine Spongiform Encephalopathy).

Another problem with the use of xenogenic tissue is the potential for inflammation responses, hematomas, adhesions, and rejection after implantation. This is because xenogenic collagen includes antigens, such as telopeptides, and other constituents that can initiate an immunogenic response in humans.

Thus, there is a need for methods to isolate collagen fibers and/or threads for products made from the collagen fibers and/or threads that are less likely to produce an immunogenic response.

### SUMMARY

Various embodiments of the invention address the issues described above by providing collagen-based medical implants suitable for implantation into humans that are derived from human or human-like collagen. The collagen-based medical implants may include one or more of the following: growth factors and other non-collagenous proteins, a low immunogenicity, and desirable handling properties.

In some embodiments collagen implants may be formed from collagen products having a preserved amount of native human or human-like constituents. Such collagen products may include collagen fiber, fibrillar collagen, microfibrillar collagen, particulate collagen, collagen thread, intermediate collagen products that may or may not contain alcohol, and that may or may not be derived from a foam containing collagen and a leveling agent. Collagen implants may include collagen films, collagen coatings, collagen strands and fabrics produced from the strands, injectable collagen, collagen tubes, collagen plugs, collagen for *in vitro* applications, collagen scaffolds, and combinations and variations thereof.

In one embodiment, a method for forming a medical implant includes blending a dispersion of human or human-like collagen product fibers and/or threads and a volume between about 2% to about 15% of an alcohol having a purity of about 70% to about 99.999%; reconstituting a foam component of the blended collagen product dispersion into a liquid phase; and removing the liquid component of the reconstituted collagen product dispersion.

In another embodiment, a method for forming a medical implant includes removing a liquid component from an intermediate collagen product to form collagen products including: films, coatings, strands and fabrics produced from the strands, tubes, plugs, scaffolds, and collagen products for injection and *in vitro* applications, and combinations and variations thereof.

Other features and advantages of the invention will become apparent from the following detailed description, taken in conjunction with the accompanying drawings, which illustrate, by way of example, various features of embodiments of the invention.

### DESCRIPTION OF THE DRAWINGS

Figure 1A depicts a method for preparing a human or human-like collagen product from harvested human or human-like tissue that may be employed according to certain embodiments of the invention.
Figure 1 B is a photograph of human fascia, which may be used as a starting material for preparing a human collagen product.
Figure 1C is a photograph of human-derived collagen product fibers and/or threads made from human fascia that is or may be prepared for use as a medical implant in accordance with certain embodiments of the present invention.
Figure 1D is a photograph of human-derived fibrillar collagen product made from human fascia that is or may be prepared for use as a medical implant in accordance with certain embodiments of the present invention.
Figure 1 E depicts methods for recovering a human-derived collagen product from human tissue according to certain embodiments of the present invention.
Figures 2A-E depict methods of forming an intermediate collagen product using collagen fibers and/or threads.
Figure 2F is a photograph of bovine-derived collagen dispersion and human-derived collagen dispersion.
Figure 2G is a photograph of a human-derived collagen product dispersion produced according to certain embodiments of the present invention.
Figures 3A-B depict methods of forming collagen products using an intermediate collagen product.
Figure 3C is a photograph of a human-derived collagen product film made from human fascia that may be prepared for use as a medical implant in accordance with certain embodiments of the present invention.
Figure 3D depicts a method of forming a collagen product using an intermediate collagen product.
Figure 3E is a photograph of a human-derived collagen product strand made from human fascia that may be used or prepared for use as a medical implant in accordance with certain embodiments of the present invention.
Figure 3F is a photograph of another human-derived collagen product strand made from human fascia that may be used or prepared for use as a medical implant in accordance with certain embodiments of the present invention.
Figure 3G is a photograph of another human-derived collagen product strand made from human fascia that may be used or prepared for use as a medical implant in accordance with certain embodiments of the present invention.
Figures 3H-J depict methods of forming collagen products using an intermediate collagen product.
Figure 3K is a photograph of a human-derived collagen product plug made from human fascia that may be prepared for use as a medical implant in accordance with certain embodiments of the present invention.
Figure 3L is a photograph of precipitated human-derived collagen product made from human fascia that is prepared for use as a medical implant in accordance with certain embodiments of the present invention.
Figures 4A-B depict methods of forming collagen product scaffolds.
Figure 4C depicts a method for forming an altered collagen product scaffold.
Figures 4D-G are photographs of collagen product scaffolds produced according to certain embodiments of the invention made from human fascia that may be prepared for use as a medical implant in accordance with certain embodiments of the present invention.
Figure 5A is an illustration of a collagen product sponge before compression.
Figure 5B is an illustration of a collagen product sheet after compression.
Figure 6 is an illustration of a wound repair dressing constructed from a human or human-like collagen product in accordance with an embodiment of the present invention.
Figure 7 is an illustration of a non-woven collagen product fabric.
Figure 8 is an illustration of a woven collagen product fabric.
Figure 9 is an illustration of a meniscus or cartilage repair structure formed using a human or human-like collagen product in accordance with an embodiment of the present invention.
Figure 10 is an illustration of a prosthetic coated with a human or human-like collagen product in accordance with an embodiment of the present invention.
Figure 11 is an illustration of an implantable instrument coated with a human or human-like collagen product in accordance with an embodiment of the present invention.
Figure 12 is an illustration of a film formed with a human or human-like collagen product in accordance with an embodiment of the present invention.
Figure 13 is an illustration of a vascular graft formed with a human or human-like collagen product in accordance with an embodiment of the present invention.
Figure 14 is a photograph, taken at 100x magnification by scanning electron microscopy, of a collagen product sponge made from human fascia that may be prepared for use as a medical implant in accordance with certain embodiments of the present invention.
Figure 15 is a photograph of a human-derived collagen product matrix prepared from intermediate collagen product II made from human fascia that may be prepared for use as a medical implant in accordance with certain embodiments of the present invention.
Figure 16 is a photograph of a human-derived collagen product matrix prepared according to known methods.
Figure 17 is a photograph of a collagen product matrix prepared according to certain embodiments of the present invention.
Figures 18A-B are photographs of a detailed portion of the collagen product matrix shown in Figure 16.
Figures 19A-B are photographs of a detailed portion of the collagen product matrix shown in Figure 17.
Figures 20A-D are scanning electron microscope (SEM) photographs of a surface of a compressed collagen product matrix provided in accordance with certain embodiments of the present invention.
Figures 21A-D are scanning electron microscope (SEM) photographs of another surface of a compressed collagen product matrix provided in accordance with certain embodiments of the present invention.

### DETAILED DESCRIPTION

Collagen is a connective tissue found in a variety of organisms, including humans and other mammals, aquatic species, avian species, etc. Collagen accounts for approximately 30% of the human body, and at least 26 collagen types in the human body are presently known, each adding specific function(s) to the collagen's structural role as connective tissue. For example, type I collagen found in tendons and the pericardium, type III collagen is found in intestines, type I or III collagen is found in fascia, i.e., tensor fascia lata, fascia lata, iliotibial band, and/or skin, type II collagen is found in cartilage and trachea, and type V collagen is found in interstitial tissue and placental tissue. In one example of the present invention, human fascia including type I collagen, type III collagen and/or elastin may be used as starting collagen material. In another example, human skin, pericardium, tendon, intestinal tissue, bladder wall tissue, placenta, etc., may be used as starting material. Collagens are described in Robert E. Burgeon and Marcel E. Nimi, Collagen Types Molecular Structure and Tissue Distribution, 282 Clinical Orthopaedics and Related Research 250-272 (1992). Fascia, a collagen-containing tissue, is described in Kathleen A. Derwin et al., Regional variability, processing methods, and biophysical properties of human fascia lata extracellular matrix, 84 J. Biomed. Mater. Res. A 500-07 (2008); Ken Nakata et al., Reconstruction of the lateral ligaments of the ankle using solvent-dried and ganema-irradiated allogenic fascia lata, 82 J. Bone Joint Surg. 570-82 (2000); and Jason Hodde, Naturally Occurring Scaffolds for Soft Tissue Repair and Regeneration, 8 Tissue Engineering 295-308 (2002).

For purposes of the present invention, the following collagen-related terms are used as follows. "Collagen" is a collagen molecule, which may contain various levels of cross-linking, or a material that is made of approximately pure or native collagen fibers or molecules. A "collagen product" is a medical product containing collagen, but which may also contain other extracellular matrix constituents (e.g., proteins such as noncollagenous proteins, including growth factors, bone morphogenic proteins (BMPs), etc.), but is processed to exclude cells that are naturally found in the collagen from the tissue source. Collagen products may be scaffolds, fibrils, particles, strands, matrices, sponges, foams, etc., or any other suitable form. "Purified collagen products" are medical products containing essentially pure collagen and are largely devoid of other natural extracellular matrix components. "Collagen-containing tissue" is a tissue sourced from the fascia lata, placenta, etc., which contains collagen, but which may also contain cells and other extracellular matrix components.

The present invention discloses methods for preparing human-based or human-like collagen products including fibers and/or threads and for producing collagen fibers, fibrils, particles, threads, strands and other implants that use human-based or human-like collagen products, so that when implanted, no or a low immunogenic response in humans results. Human-like collagen is collagen derived from a non-human source that may be treated to result in a collagen product that is implantable and produces no or a low immunogenic response in humans. Human-like collagen may be transgenic or genetically engineered collagen and may be enzymatically treated to remove immunilogically active gylcoproteins and recombinant collagen. The collagen-containing medical implants provided according to some embodiments have one or more of the following attributes, including physiologically compatible, sufficiently noninfectious to prevent transmission of viruses and prions and growth of bacteria (vegetative and spores) and fungi, pliable, available for a wide variety of applications in a variety of shapes and sizes, high in tensile strength, and inert.

According to various embodiments of the invention, any of a variety of types of human connective tissue and connective tissue from other organisms including genetically engineered animals may be processed to yield human or human-like collagen products. Collagen products provided according to some embodiments may be supplemented with cells and/or proteins such as stem cells. Accordingly, collagen products provided according to aspects of the invention may include collagen with non-collagenous proteins and/or extracellular matrix, which may or may not be supplemented with cells not naturally present in the source collagen tissue.

### Preparing Collagen Fibers and/or Threads

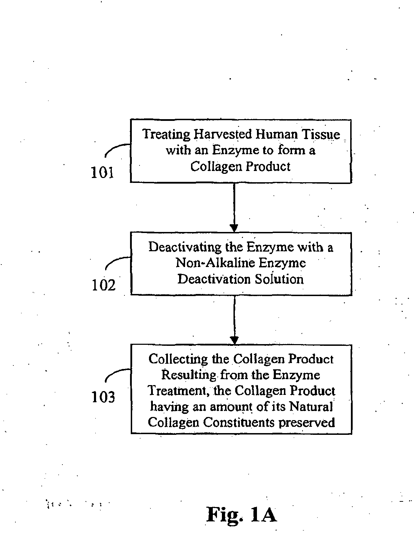

Figure 1A depicts a method for preparing human-derived or human-like collagen products from harvested human or human-like collagen-containing tissue, according to certain embodiments of the invention. The method of Figure 1A includes treating (101) harvested human or human-like collagen-containing tissue with one or more enzymes to yield a collagen product that is suitable for implantation into humans. The enzyme is deactivated (102) using a non-alkaline enzyme deactivation solution, and the collagen product resulting from.the enzyme treatment is collected (103). Where the original collagen source is human, the resulting collagen product, e.g., non-immunogenic human collagen fiber, includes a preserved amount of its native human constituents. Collagen products that contain a preserved amount of its native human constituents retains a sufficient or effective amount of the original collagen structure and/or constituents, including non-collagenous proteins and/or cross-link chemistries, to be suitable or therapeutically beneficial for its intended application.

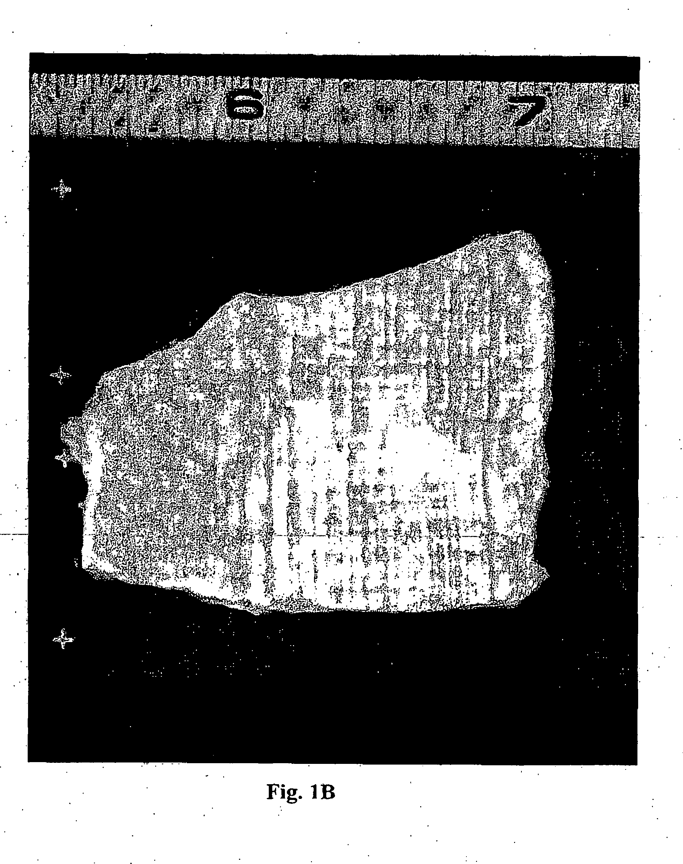

Figure 1B is a photograph of human fascia, which may be used as a starting material for preparing human collagen products according to the method of Figure 1A. The human fascia depicted in Figure 1B includes banded collagen evidenced by its vertical stripes that traverse the fascia sample. Collagen fibers bound in fascia are biologically manufactured as extra-cellular protein units (e.g., helical assemblies of amino acids) that are about 300,000 nanometers in length.

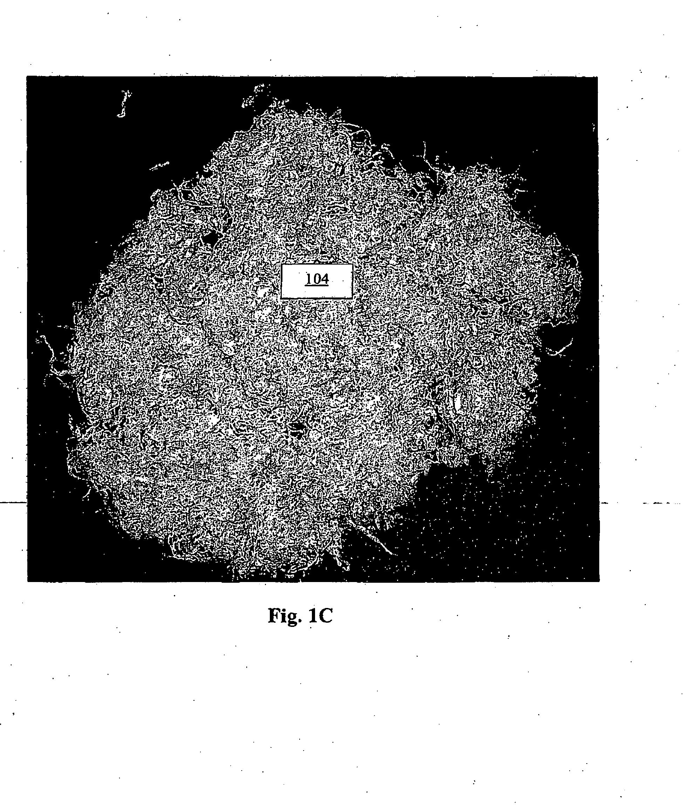

Figure 1C is a photograph of human collagen fibers and/or threads 104 that may result from processing bound collagen in human fascia according to the collagen product production method of Figure 1A. The human collagen fibers 104 may be used as or prepared for use in a medical implant in accordance with certain embodiments of the present invention. In Figure 1C, the prepared human collagen fibers and/or threads 104 appear beige in color, have a diameter of about the diameter of a human hair to about the diameter of a plant fiber, e.g., flax, a length of about 10 cm to a particulate size such as about 50 microns, with an average length of about 3.2 cm (1.25 in., e.g., common staple fiber), are coarse to the touch like coarse cotton, hemp or hair.

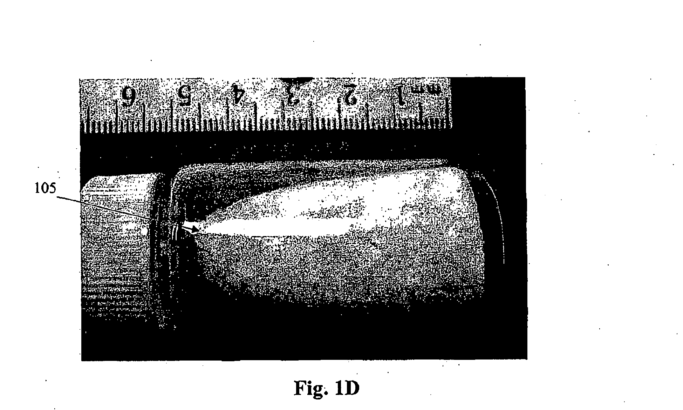

Figure 1D is a photograph of prepared fibrillar collagen 105 made from milled collagen fibers like those pictured in Figure 1C, for example. In Figure 1D, the fibrillar collagen 105 appears like a particulate, but when viewed microscopically may be fiber-like in appearance. Accordingly, the characteristics of fibrillar collagen may be similar to the collagen fibers and/or threads but for the shorter length of the individual fibrillar collagen grains and possibly smaller diameters.

The above-described method for preparing a human derived collagen product, e.g., fibers and/or threads, involves enzymatically treating by ficin treatment, harvested human tissue to separate collagen fibers and/or threads in tissue from other components and to break down peptide bonds between amino acids of proteins in the collagen, while retaining certain native constituents and receptivity of the human-derived or human-like collagen. For example, native constituents may include uniquely human or human-like biological characteristics, which allow the collagen product to be biocompatible. In some implementations, the enzyme treatment breaks down some of the telopeptide bonds, while leaving others intact. This results in partly bound collagen fibers and/or threads retaining a portion of the native non-collagenous proteins. The fibers and/or threads are non-immunogenic due to their human or human-like origins. The method of Figure 1A and additional methods for preparing human collagen products with native human constituents preserved involving the use of enzyme treatment are described in U.S. Patent Application No. 11/673,972, filed February 12, 2007, entitled "Methods for Collagen Processing and Products using Processed Collagen,". However, it will be understood that collagen products may be prepared using any known method.

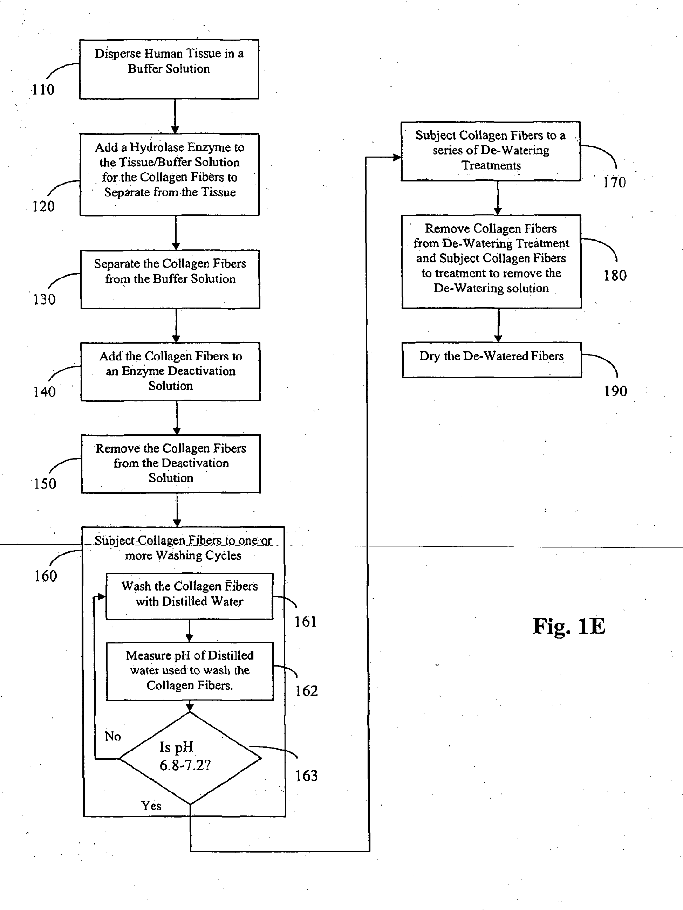

Figure 1 E depicts a more detailed collagen product preparation method according to certain embodiments of the present invention. According to one method, finely ground or sliced human collagen-containing tissue (such as fascia, tendon, and/or small intestine submucosa) containing bound collagen is dispersed (110) in a buffer solution at a suitable temperature and pH. Any suitable buffer solution at any appropriate pH and temperature may be used for providing an environment for the efficient use of a particular enzyme to enable the enzyme to attack and remove material. In the exemplary use of ficin in a buffer solution of potassium phosphate (KH₂PO₄) and sodium hydroxide (NaOH), enzymatic activity is carried out efficiently at a pH of 6.3 +/-0.15 and at a temperature of 37 °C +/- 1.5 °C. However, it will be understood that buffer solutions may be suitable at any appropriate pH, such as a pH from about 3 to about 9, from about 5 to about 7, or from about 6.0 to about 6.3. Further, buffer solutions may be suitable at any appropriate temperature such as between about 20 °C and about 50 °C, between about 30 °C and about 40 °C, or about 37 °C. After the collagen-containing tissue is added to a buffer solution, a hydrolase enzyme is added (120). The hydrolase enzyme is ficin. The hydrolase enzyme assists in catalyzing the cleavage of proteins and solubilizing other tissue components and non-collagenous impurities. The enzyme may be kept in solution for an appropriate amount of time for the enzymatic activity to cause telo-peptide bonds to be broken down, which may allow the collagen fibers to unwind, as evidenced by the appearance of strand-like collagen in solution. Any suitable length of time may be used, including time ranging from seconds to minutes to hours or longer. For ficin, the enzymatic activity occurs for about 30 minutes with intermittent stirring. However, the amount of time the enzymatic activity the tissue in solution undergoes may be adjusted so that the collagen fibers from the collagen-containing tissue preserve their fiber orientation and/or native constituents that may provide potential benefits. For example, by preserving the original or native constituents in human-derived collagen products, an implant may provide that, when implanted, produces no or a low immunogenic response and allows implants to disperse and/or crosslink after implantation. In addition, retaining components of the extracellular matrix in the collagen product may promote healing.

The enzyme-treated collagen fibers are separated (130) from the enzyme-buffer solution and added (140) to a non-alkaline enzyme deactivation solution selected based on ficin. A suitable deactivation solution may be sodium chlorite (NaClO₂) in an ammonium nitrate (NH₄NO₃) buffer solution. Alternatively, the deactivation solution may be an oxidizing agent such as hydrogen peroxide in a sodium chlorite buffer solution. In addition, use of an oxidizing agent may also facilitate in bleaching the fibers. The collagen is exposed to the deactivation solution for an amount of time sufficient to deactivate the enzyme reaction, for example about 1 hour when the enzyme is ficin. The enzyme deactivation solution will be a non-alkaline solution, which may be less harsh on the fibers, thereby assisting in retention of the natural collagen product constituents, e.g., collagen, extracellular protein constituents, but excluding tissue-source-derived cells.

The treated fibers are removed (150) from the deactivation solution and subjected (160) to a series of washing cycles. Each washing cycle involves washing (161) the fibers with a suitable amount of liquid, such as about 500 ml distilled water, for a suitable period, such as about 15 minutes. The collagen product is compressed to squeeze out excess water and the pH of the distilled water used in washing the fibers is taken after each wash period (162). The pH after the first and second wash is expected to be about 7.0 +/- 0.5, and after a third wash is expected to be about 7.0 +/- 0.2. Although three washes of the fibers are described in the present embodiment, it will be understood that when the pH of the distilled water reaches a desired pH range, e.g., about 7.0 +/- 0.2, the washing process may be terminated. It will be understood that any suitable pH range can be used for this purpose, including from about 3 to about 9, from about 5 to about 7, or from about 6.0 to about 6.3.

In one embodiment, after washing with distilled water, excess water may be removed from the washed fibers by any suitable method, such as compression or squeezing. For example, fibers may be hand squeezed, pressed onto a fine screen, vacuumed, centrifuged, combinations thereof, etc. Optionally, the fibers may undergo (170) a series of de-watering treatments. Any suitable treatment may be used, including, by way of example only, placing the fibers into a bath of about 100% isopropanol (IPA), heating to about 60 °C, and blending for about 15 to about 60 seconds. The fibers may remain in the de-watering solution as appropriate, including for about 2 hours at about 60 °C, optionally with intermittent stirring. After the first de-watering treatment, the fibers may be separated from the solution, squeezed, and subjected to another de-watering treatment, as desired. The subsequent de-watering cycle may be repeated in the same manner. In various embodiments, the time spent by the fibers in the de-watering solution may vary. For example, in subsequent de-watering steps, the fibers may remain in the de-watering solution for about one hour as opposed to about two. In the exemplary use of about 100% IPA as the de-watering solution, the IPA, in addition to removing water from the fibers, also may assist in the removal of any oils present in the collagen product mixture.

After the de-watering cycles, the fibers are transferred (180) to another bath for removing the de-watering solution. For example, when IPA is the de-watering solution, the fibers may be added to an about 100% acetone bath and heated to about 40 °C. In addition, the fibers in the bath may be blended for a period of about 15 to about 60 seconds. Removing the de-watering solution with about 100% acetone, in addition to removing alcohols or water, also may remove any oils potentially present in the collagen product mixture.

The purified fibers may be removed from the bath, separated apart from each other, and dried (190) as appropriate. One suitable drying procedure includes drying at about 40-45 °C for a period of time, such as about 4-12 hours, although any other suitable drying procedure also may be used. The isolated, enzyme treated human collagen fibers in particular embodiments includes natural, native collagen constituents, and may be used for a variety of applications including for medical implants.

The collagen production and purification method may be supplemented or steps may be altered to preserve a desired collagen product. For example, the collagen preparation process may include a terminal sterilization procedure that may include dialysis, irradiation, filtration, chemical treatment, or other suitable procedure. In addition, collagen or tissue-containing collagen may be blended at various other points in the recovery process in addition or as an alternative to the blending processes described above. Further, homogenizing the collagen mixture may replace or supplement blending. Moreover, in order to further express water from the fibers after washing with distilled water or after the de-watering step, the collagen fibers may be frozen so that any remaining water is expelled.

The collagen preparation methods of the present application may result in human collagen fibers that are relatively pure, *e.g*., greater than about 70%, greater than about 80%, greater than about 90%, greater than about 95%, or greater than about 98%. According to the embodiments of the present invention, purified collagen fibers means that the fibers are treated, cleansed, or made suitable for implantation and for use as medical devices using any suitable collagen preparation, preservation, recovery or purification methods, including the methods described above. Purity does not denote any particular degree of purity, and may include a variety of levels of purity, as appropriate for the intended purpose.

In some embodiments, the collagen recovery and collagen product preparation method of the present invention does not use an alkali treatment step, and a non-alkaline solution is used for enzyme deactivation. This is useful according to embodiments of the present invention because certain collagen constituents native to humans, *e.g*., human growth factors and morphogenic proteins that would otherwise be stripped away by exposure to an alkaline solution, are maintained. In addition, because the collagen fibers are derived from humans, harsh purification and/or treatment processes may be unnecessary because human based collagen-containing tissue is less likely to be contaminated as compared to xenogenic collagen-containing tissue. It will be understood that collagen product preparation may be accomplished using a variety of methods and may include collagen processing steps in addition to or as an alternative to the processing steps described above.

Moreover, because the collagen fibers are sourced from humans, collagen products formed from these fibers are less likely to produce an immunogenic response when used for implantation into humans. Accordingly, the human collagen recovery and collagen product production methods, according to certain embodiments of the present invention, are simplified method compared to xenogenic collagen recovery methods, and end products made from the human derived collagen products are desirable, as they are likely to be accepted at an implant site.

Other collagen.product preparations methods may also be employed according to embodiments of the invention. For example, harvested collagen-containing tissue may be scraped, sliced, e.g., from frozen specimens, lyophilized, and/or treated enzymatically, etc., to yield collagen products including fibers, fibrils, microfibrils, particles, threads, strands, etc.

Prepared collagen products may be stored in fiber, fibrillar (e.g., milled fibers), microfibrillar (e.g., appear like a fiber when viewed microscopically) and/or particulate form (e.g., ground collagen). Such prepared collagen products may be suitable for medical use in humans in their native form. Fibrous, fibrillar, microfibrillar and/or particulate collagen products in their native form may be useful as a hemostat in applications such as general surgery and/or to treat injuries, e.g., for emergency field treatment or other treatment..

Alternatively, collagen products may be processed into another, form of a medical implant. Because the collagen product retains a portion of its collagen constituents that remain at least partly bound to each other and retain a portion of native non-collagenous proteins, implants may be non-immunogenic (e.g., due to the human or human-like origin), and may have improved elasticity and strength characteristics (e.g., resistant to cracking) compared to collagen implants derived from other sources (e.g., bovine-derived collagen).

### Intennediate Collagen Products Produced from Prepared Collagen Fibers and/or Threads

### Intermediate Collagen Product I: Dispersed Collagen in Solution

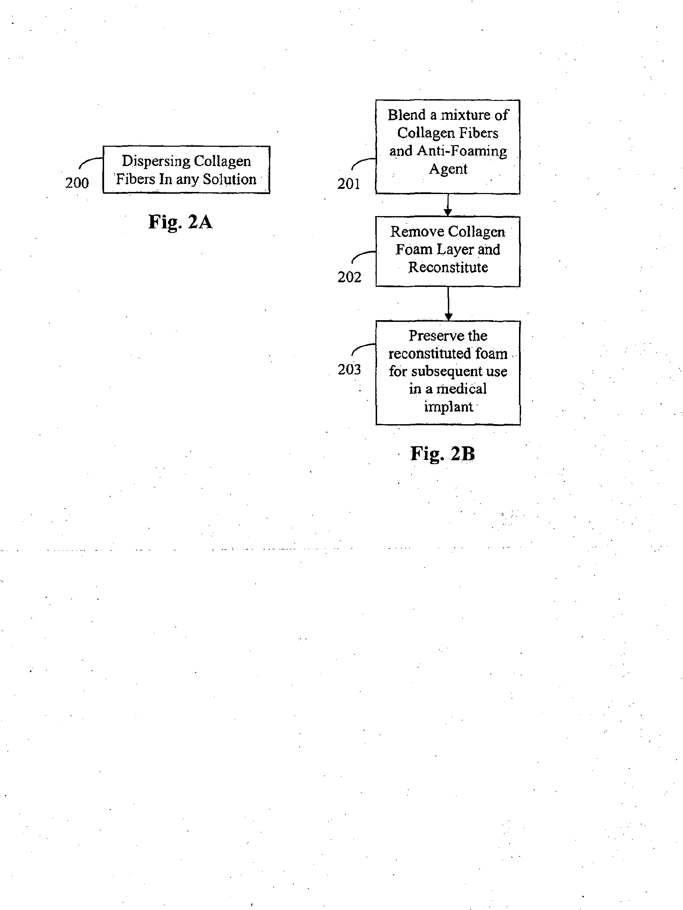

According to an aspect of the invention, collagen fibers and/or threads prepared according to the method of Figure 1A may be used as a starting material to produce an intermediate collagen product I. In Figure 2A, the intermediate collagen product may be provided by dispersing (200) the prepared collagen in any suitable solution including a distilled water and lactic acid solution, or a buffer solution at any suitable temperature and pH.

### Intermediate Collagen Product II: Foam containing Collagen and a Leveling Agent

Intermediate collagen product II may be provided according to the method of Figure 2B in which a mixture of dispersed collagen fibers and/or threads and a volume of a leveling agent (e.g., an alcohol that is about 0.25% to about 15% by volume having a purity of about 50% to about 99%) are blended (201) resulting in a foam containing at least human-derived collagen and the leveling agent. The foam may be removed and reconstituted (202) as desired. For example, the foam may be reconstituted into a liquid phase such as by changing the gas-containing foam into a substantially gas-free liquid by centrifuging the foam at about 1500 to about 3000 rpm for about 1 to about 5 minutes. The collagen-containing foam reconstituted into a liquid is an intermediate product that may be preserved (203) for subsequent use in medical implant production processes. In the present invention, intermediate collagen product II is one or both of a foam containing collagen and a leveling agent (e.g., alcohol) or its reconstituted liquid. Medical implants having improved properties may be produced using such an intermediate product containing and are described in relation to collagen products below.

According to the method of Figure 2B, when a leveling agent is blended (201) with the collagen dispersion, a separation occurs leaving a foam layer on top of a flowable liquid. The resulting foam may be separated and reconstituted (202) into a liquid. The foam layer is believed to consist of one or more types or constituents that may be different from the flowable liquid component because collagen products formed from the foam is more firm and hard compared to the collagen products from the flowable liquid. While not desiring to be bound to any particular theory, such physical characteristics may indicate that the foam constituents may include collagen that is feral/native collagen having bonds (telopeptide and carbohydrate) that have not been broken or removed, or may be a specific type or types of collagen, e.g., elastin and type III collagen, and/or may be indicative of additional components such as non-collagenous proteins, e.g., growth factors.

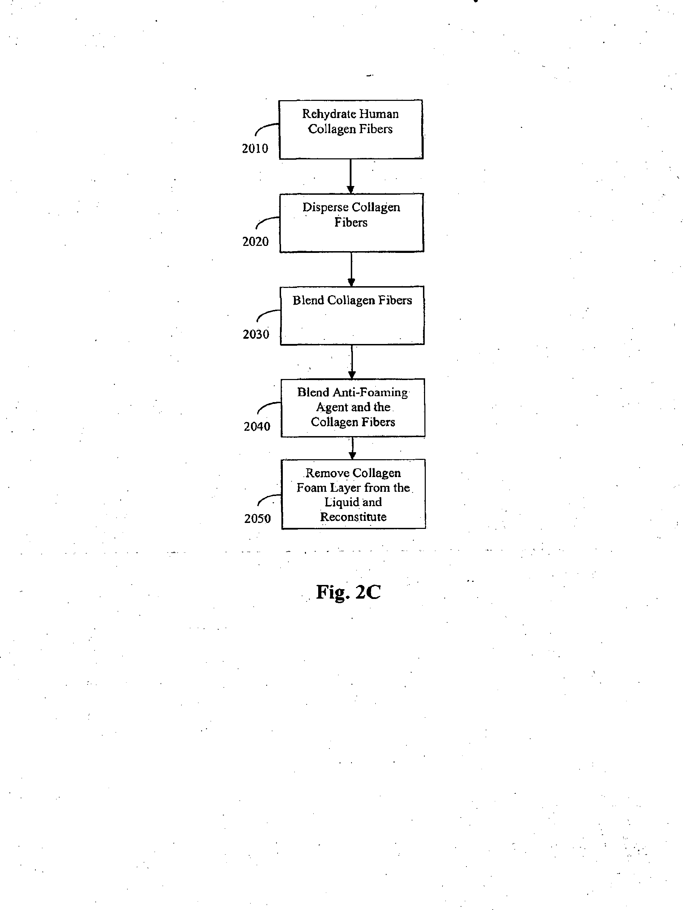

The intermediate collagen product II may also be formed using the method of Figure 2C in which a collagen product of prepared human-derived or human-like collagen fibers and/or threads are hydrated (2010), dispersed (2020) by blending (2030) or homogenizing, blended with a leveling agent (2040), and the foam removed and reconstituted (2050). Collagen fibers and/or threads are hydrated (2010) by adding dehydrated or dried collagen fibers and/or threads to a media that allows the fibers and/or threads to become swollen and take up water without denaturing the triple helix structure of the collagen. Any suitable media may be used, including an acidic media. One example of an acidic dispersing media that is suitable for dispersion of the human collagen fibers and/or threads and their resulting rehydration when forming a dura/meningeal repair matrix is an about 85% lactic acid solution in distilled water at a ratio of about 1:500, where the collagen fibers and/or threads are permitted to swell for about 1 hour at a temperature of ≤ about 15 °C. Any of these parameters may be adjusted as desired for the particular application. The reconstituted collagen product in solution may have an about 0.5 to about 1.25% collagen density, or an about 0.75% collagen density, although any other values can be used, as appropriate.

After reconstitution, the collagen product dispersion is prepared (2020) by any suitable method. "Dispersion" used in the present application encompasses any type of dispersion method including blending, mixing, agitating, and/or suspending in a mixture of water, water and an acid, e.g., lactic acid. One example of a suitable dispersion preparation method includes blending (2030) or homogenizing the fibers and/or threads in solution having a preferred temperature of about 10 to about 40 °C, about 10 to about 35 °C, or about 10 to about 20 °C, at various speeds for intervals of about 5 to about 25 seconds, with a time period of about 10 to about 60 minutes between blending intervals. In a particular example, a blending series includes blending at low, e.g., about 14,000 to about 16,000 rpm, medium, e.g., about 16,000 to about 19,000 rpm, and high speeds, e.g., about 19,000 to about 22,000 rpm, for about 10 seconds, with an interval of about 30 minutes between each blending speed, and is repeated about three times. Any of these parameters may be varied as dictated by the fiber and density specified by the product under construction. According to the presently described embodiment, the resulting dispersion may have an about 0.75% collagen density at a pH of about 2.8 to about 3.2, though any desired density and pH may be achieved.

The blended dispersion may be mixed with a leveling/precipitating agent and blended (2040) in intervals, e.g., low, medium, and high speed blending with about 30 minutes between intervals. The leveling/precipitating agent cause the collagen to at least partly precipitate in the solution. Such leveling/precipitating agents may include polyhydroxy compounds (e.g., ketones such as acetone), alcohols (e.g., ethyl alcohol (EtOH), isopropanol (IPA), surfactants, salts, etc. In one example, an alcohol having a purity of about 60% to about 99%, about 70% to about 99%, about 90% to about 99%, or greater than about 99%, at a concentration between about 0% to about 15% by volume, between about 3% to about 6% by volume, or about 5% by volume (e.g., EtOH having a purity of about 70% to about 99% at a concentration of about 5% EtOH by volume) may be added to the blended dispersion. Alternatively isopropanol (IPA), e.g. about 60% to about 99% pure IPA, may be used alone or in combination with EtOH. Other polyhydroxy compounds are disclosed in U.S. Patent No. 5,290,558, issued on March 1, 1994, entitled "Flowable demineralized bone powder composition and its use in bone repair." Moreover, in addition to or as an alternative to the precipitating mechanisms, dewatering mechanisms are also contemplated which dehydrate collagen in solution causing the collagen to separate from water.

The resulting dispersion includes a foam layer on top of a liquid or fluid layer, each of which may contain a precipitated amount of collagen. Any resulting foam is removed and reconstituted (2050) into a gas free liquid phase, for example, by decanting the fluid from the foam, collecting the foam, and centrifuging the foam, e.g., at about 2500 rpm for about 1 to about 5 minutes.

When the leveling agents discussed above are used in preparing collagen product suspensions for collagen derived from non-human sources, foam is typically reduced or eliminated. That is, a leveling agent for a human-sourced collagen product would be an anti-foaming agent for a non-human sourced collagen. For example, upon blending an alcohol, such as ethanol, with a bovine collagen suspension, foam is typically reduced or eliminated. In the present invention, it has been discovered that by adding a component traditionally believed to be an anti-foaming agent and blending with a collagen product, a leveling effect is instead produced, and leveling agents for human-derived collagen products are not leveling agents for non-human-derived collagen. Where a leveling agent such as EtOH is used, the resulting product (e.g., sponge matrix) may be less susceptible to cracking during lyophilization, may be more homogeneous (with no or fewer fault lines that could be susceptible to tearing), and may have a crystal formation that is more regular, with less sharding.

### Intermediate Collagen Product III: Collagen Containing a Leveling Agent

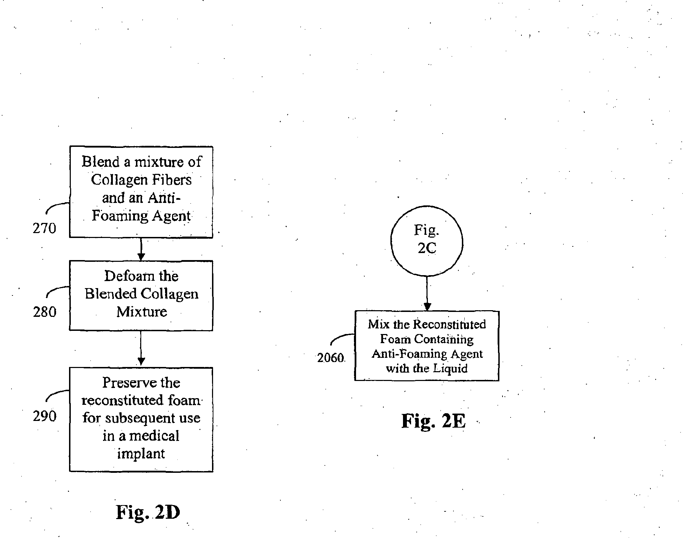

Intermediate collagen product III may be provided according to the method of Figure 2D in which collagen fibers and/or threads are dispersed in a suitable water, lactic acid, and leveling agent mixture and blended (270), and the resulting foam/liquid mixture defoamed (280) using any known method, such as by adding defoaming agents including surfactants, soaps, alcohols, tension reducing materials that are acceptable to biological activity or that are removed in processing, by mechanical means including mixing platforms that do not form surface foams (e.g., airless static, planetary Ross or Lee mixers, Graeco in line airless), or by foam elimination using an ultrasonic or vacuum-break processes. The defoamed collagen product mixture is preserved (290) for subsequent use in forming a medical implant. Accordingly, the intermediate collagen product in the present case includes all of the collagen product originally dispersed mixed with a volume of a leveling agent.

Other intermediate collagen product production methods mayalso be employed. For example, intermediate collagen product III may be produced by reconstituting, dispersing, and blending a collagen product, separating the collagen product's collagen-containing foam and reconstituting, filtering, degassing, and/or centrifuging the dispersion separate from the foam, remixing the foam component, and/or reincorporating the collagen components, e.g., the collagen product components including the collagen dispersion and the collagen-containing foam. Intermediate collagen product III may also be provided according to Figure 2E, which includes the method of Figure 2C plus the introduction of the reconstituted foam component into the decanted collagen fluid and mixing (2060) to form a homogenous collagen product mixture. In some implementations, the homogenous mixture is refrigerated, e.g., at about 4 to about 10 °C, for about 3 to about 24 hours before undergoing further processing. In further implementations, the decanted collagen fluid is refrigerated before the foam component is re-mixed to form a homogenous mixture.

The third intermediate collagen product may provide various advantages due to its leveling agent (e.g., alcohol) content and due to its retention of all of the originally dispersed collagen product. Certain advantages are provided further below in relation to the collagen product scaffold production methods that involve freezing the collagen product dispersion.

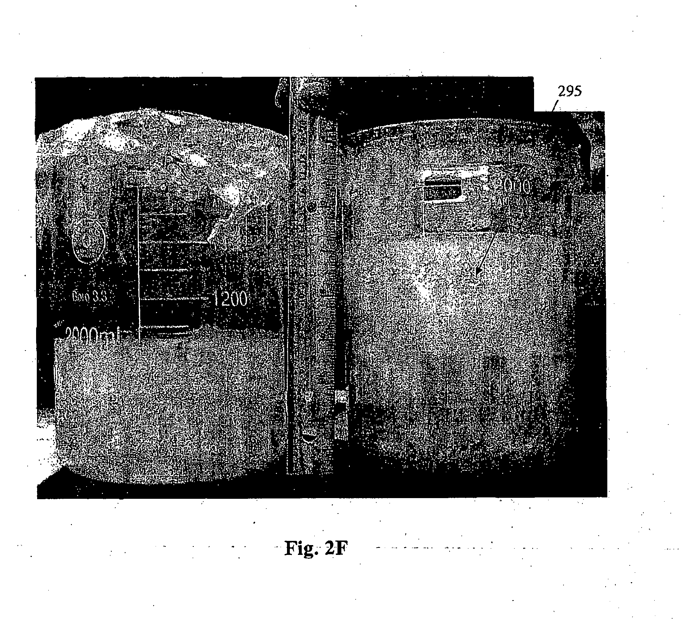

The methods described above in relation to intermediate collagen products II and III differ from other collagen product production and processing methods because typically leveling agents for human-derived collagen product are not leveling agents for non-human-derived collagen, and the type of foam produced when blending human-derived collagen product with a leveling agent is not produced upon blending collagen derived from other non-human-like sources (see Figure 2F, discussed below). Even where foam is produced in human-derived or non-human-derived collagen product, it would typically be considered waste and discarded, while the liquid homogenous phase would be retained for further use. This is because the foam: 1) is not homogeneous with the rest of the dispersion, 2) is not a typical result when blending other non-human forms of collagen with alcohol, 3) is persistent and does not dissolve into solution unless manipulated mechanically and/or chemically, 4) may contain a relatively small amount of the dispersed collagen and thus be easily discarded without affecting the batch size, and/or 5) may be easily removed by pouring and employing a weir or spatula.

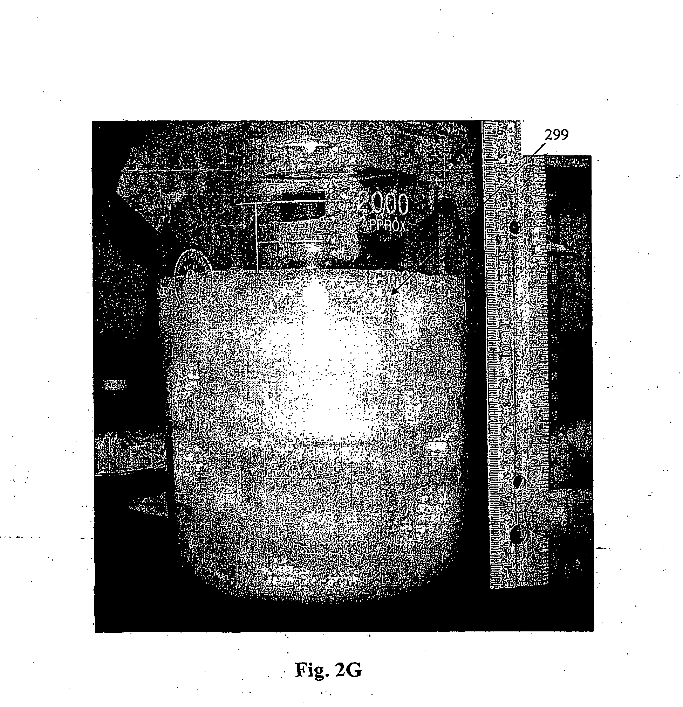

Each of the above-disclosed intermediate collagen products I-III when in dispersion may appear to have a greenish/yellow tinge, that is slightly thickened, yet self-leveling. When the dispersion includes alcohol or another leveling/precipitating agent, mixing and/or shaking the dispersion creates a foam layer coritainiitg collagen and alcohol. Figure 2F is a photograph of bovine-derived collagen dispersion (left) and human-derived collagen product dispersion (right) after blending, each dispersion having about a 0.75% collagen density in an about 1 Liter batch having about 50 ml of 99.99% EtOH and about 5 ml of 85% lactic acid. From Figure 2F it can be seen that human-derived collagen product in dispersion (right) produces a foam layer 295 when blended, whereas bovine-derived collagen in dispersion (left) does not. Figure 2G is a picture of the human-derived collagen product dispersion from Figure 2F, in which foam layer 299 having about a 6 cm depth can be more easily discerned. The human-derived collagen product foam pictured in Figure 2G is persistent foam that is sustained over time, and no observable change in the foam occurs when it is refrigerated for about a month. In addition, when the human-derived foam is permitted to dry at room temperature, a nearly transparent film is produced that is flexible and exhibits some plasticity. While not desiring to be held to any particular theories, it is believed that human-derived collagen product foam includes constituents or properties different from bovine-derived collagen at least because mixing a bovine collagen suspension does not produce persistent foam. Additional reasons human-derived collagen foam is believed to have unique constituents are discussed below in relation to producing collagen scaffolds using the foam component of a human-derived collagen suspension. Possible reasons for the differences in bovine and human collagen products include the relative age of the collagen specimen results in a different amount of cross-linking, bipedal vs. quadrapedal locomotion cause fascia to differ, differing food intake or uncontrolled substances can vary the composition of collagen-containing tissue, human collagen-containing tissue may be affected by different diseases, weight is controllable for bovine samples, and bovine samples have increased growth hormones.

Various medical implants may be constructed using any of the intermediate collagen products described above, and include: films, coatings, drug delivery devices, woven structures, mesh structures, injectable substances, vascular/neural grafts, tubes, plugs, repair matrices, scaffolds, and/or hemostats. Additional medical implants that may be produced are described in U.S. Patent Nos. 6,485,723, issued November 26, 2002, entitled "Enhanced submucosal tissue graft constructs;" 7,147,871, issued December 12, 2006, entitled "Submucosa gel compositions;" 4,956,178, issued September 11, 1990, entitled "Tissue graft composition;" and 5,554,389, issued September 10, 1996, entitled "Urinary bladder submucosa derived tissue graft;" and in the article, Stephen F. Badylak, The Extracellular Matrix as a Biologic Scaffold Material, 28 Biomaterials 3587-3593 (2007).

The various implant fabrication processes described below use one or more of the intermediate collagen products to yield a collagen implant that is suitable for implantation into humans. However, it should be understood that, in some embodiments, the intermediate collagen products may be suitable as a finished product for implantation into humans without further processing.

### Medical Implants Formed from Intermediate Collagen Products I-III

### Collagen Product Films/Coatings

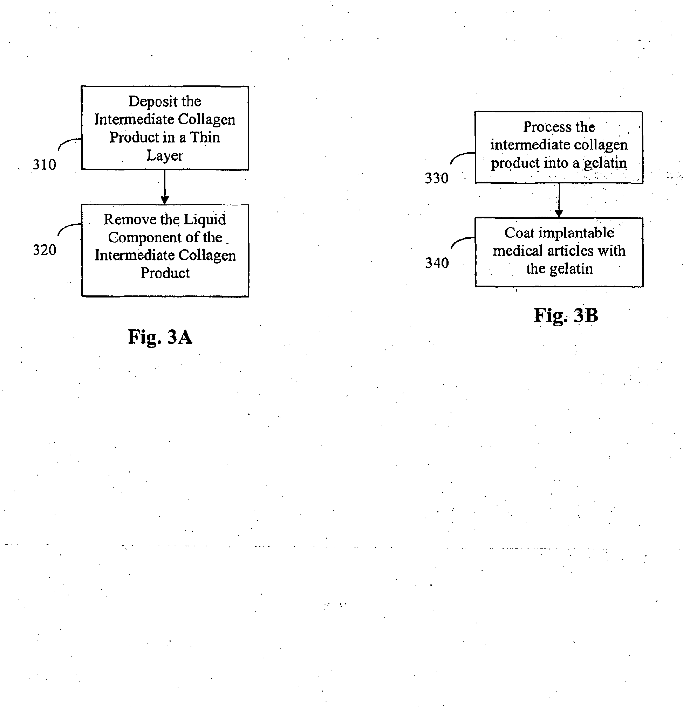

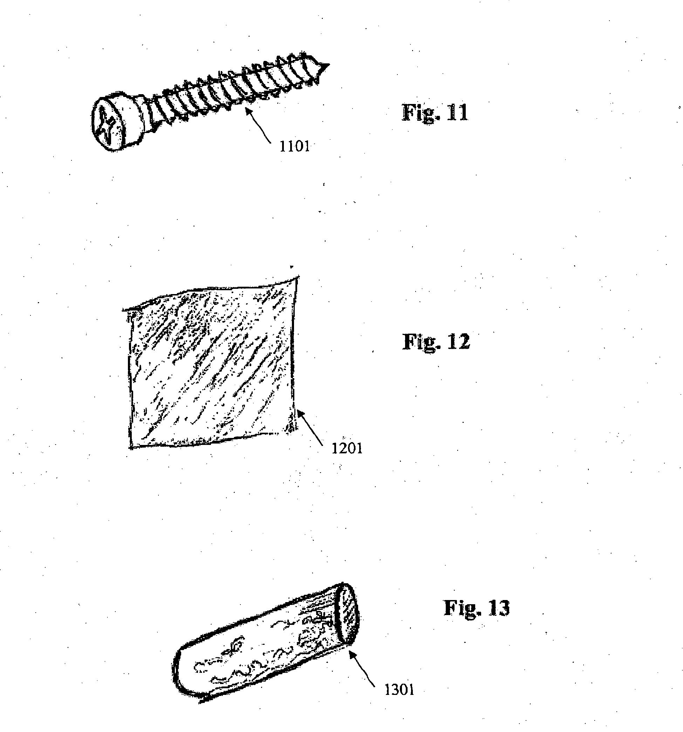

A film barrier 1201 from Figure 12, may be produced using any one of the intermediate collagen products described above. According to Figure 3A, a film barrier may be fabricated by depositing (310) the intermediate collagen product in a thin layer and removing (320) the liquid component. "Removing the liquid component" used in present application encompasses any type of moisture removal process and includes freezing and lyophilizing, lyophilizing, evaporating by heating, allowing the dispersion to remain at room temperature while the liquid component evaporates naturally, or any other suitable moisture removal process. The resulting sheet may be used as a film, or may be processed further to achieve desired characteristics. In addition, before removing liquid from the intermediate collagen product, other biocompatible materials may be mixed with the collagen suspension where certain performance characteristics are desirable.

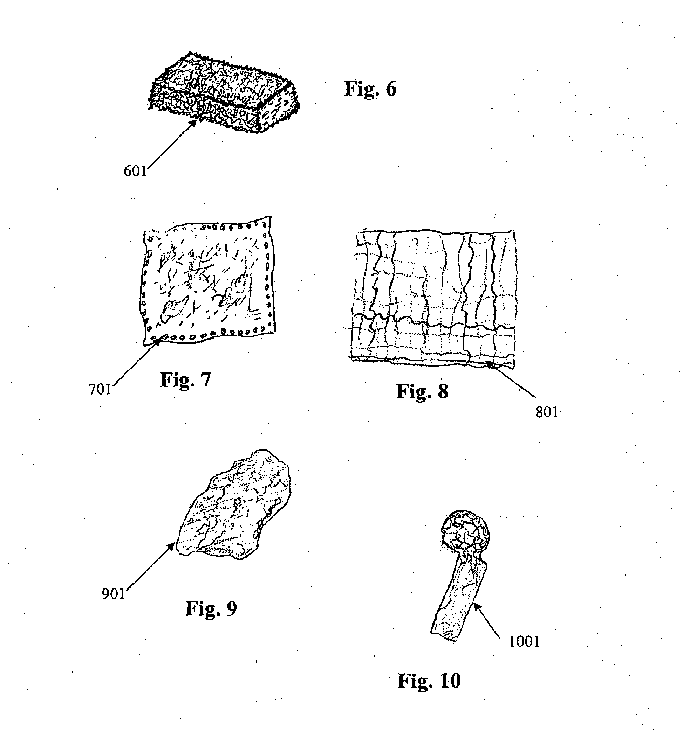

According to Figure 3B, an intermediate collagen product may be processed (330) into a gelatin, and the gelatin may be used as a coating to coat (340) medical implants. In certain implementations, various prosthetics, e.g., prosthetic 1001 in Figure 10, and/or instruments, e.g., instrument 1101 in Figure 11, may be coated with the gelatin produced from the intermediate collagen product.

Films and/or coatings may be useful, for example, in barrier dressings (e.g., adhesion barriers and barriers to liquids), occlusions, structural supports, osteochondral retainers for cells/matrices (+/- analgesic), drug delivery devices, e.g., collagen product coating combined with analgesic, anti-inflammatory, antibiotic, and/or growth factors, and wraps for bone defects. In addition, catheters and stents may be coated. In a further implementation, a plasticizer, bioactive, bioabsorbable, soluble, and/or biocompatible component may be combined with the collagen product or the gelatin formed from human-derived or human-like collagen product in order to form a collagen product paste, slurry and/or putty, etc. In a further embodiment, a collagen product gel or film may be combined with a structural backing, e.g., a thin film, e.g., about 100 to about 200 um or about 0.05 to about 0.5 mm, such as a polylactide and/or chitosan film. The collagen product coatings and/or films may provide one or more durable layers of collagen product that may be used in general medical, cardiovascular, and/or orthopaedic settings.

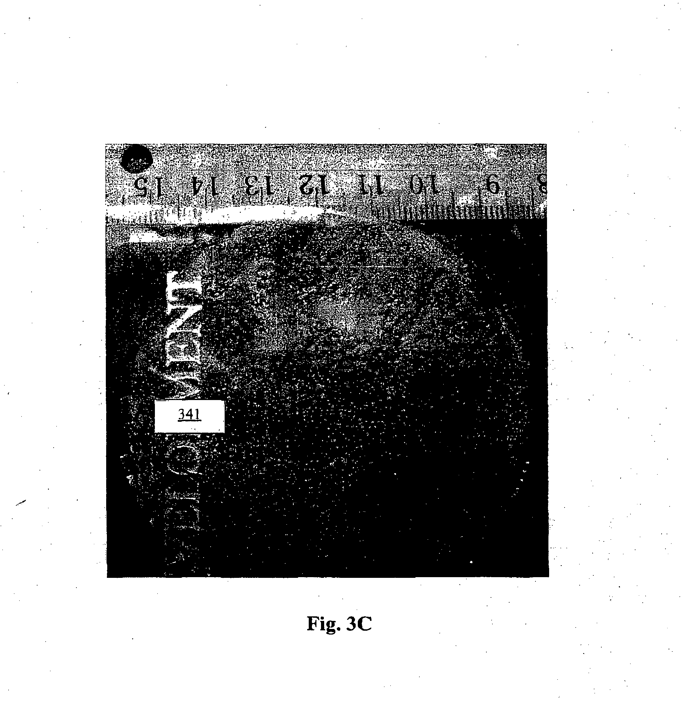

A human-derived collagen product film 341 made from human fascia is depicted in the photograph of Figure 3C, which may be prepared for medical use in humans in accordance with certain embodiments of the present invention. Exemplary physical characteristics of the collagen product film and/or coating may depend on the type of starting material and/or intermediate collagen product or products used to produce the film and/or coating, and may include: pliable, flexible, resistant to cracking, strong, and/or dense.

### Collagen Product Strands and Collagen Products Formed from Strands

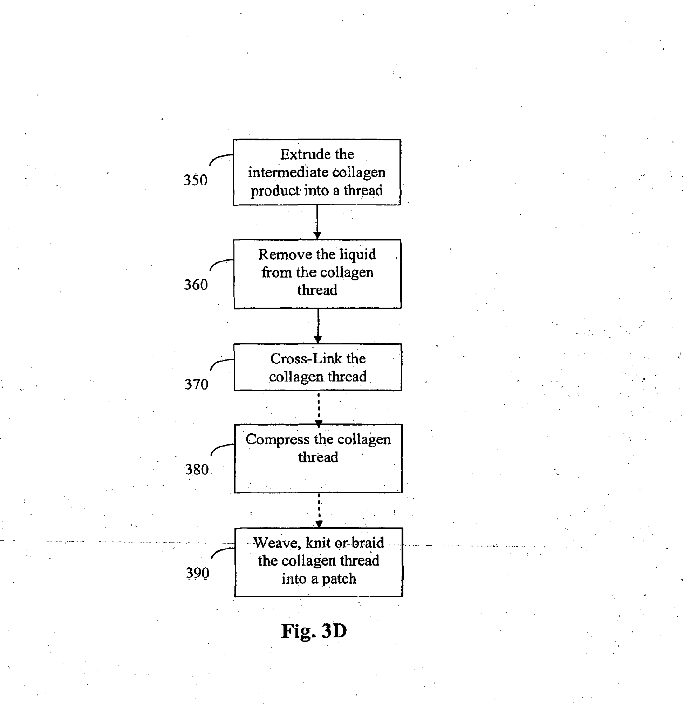

According to Figure 3D, the intermediate collagen product may be processed into a strand by extruding (350) the intermediate collagen product into a strand, removing (360) the liquid component from the collagen product, e.g., by lyophilization, and cross-linking (370) the collagen product to form a strand. In some implementations the collagen product strand may be compressed (380), woven, knitted, and/or braided (390) into a patch. Alternatively, the strand may be cross-linked in-situ during the extrusion process.

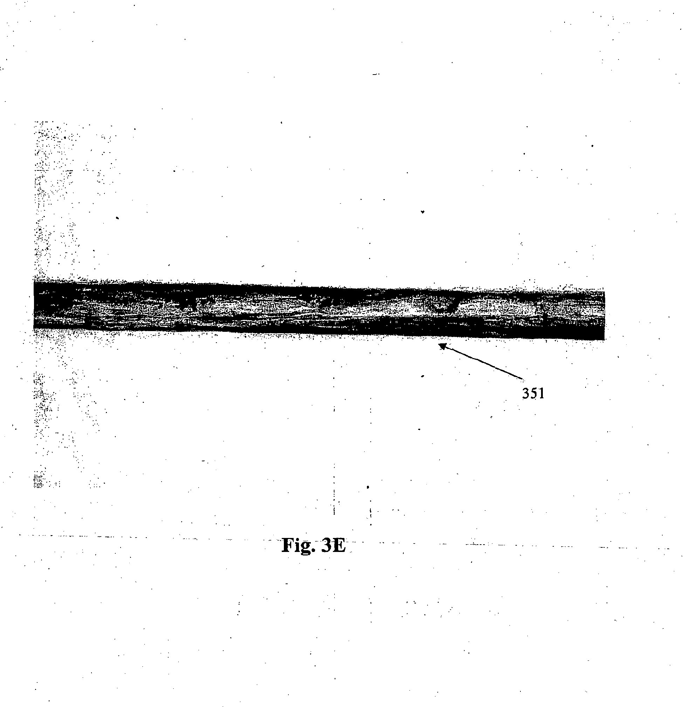

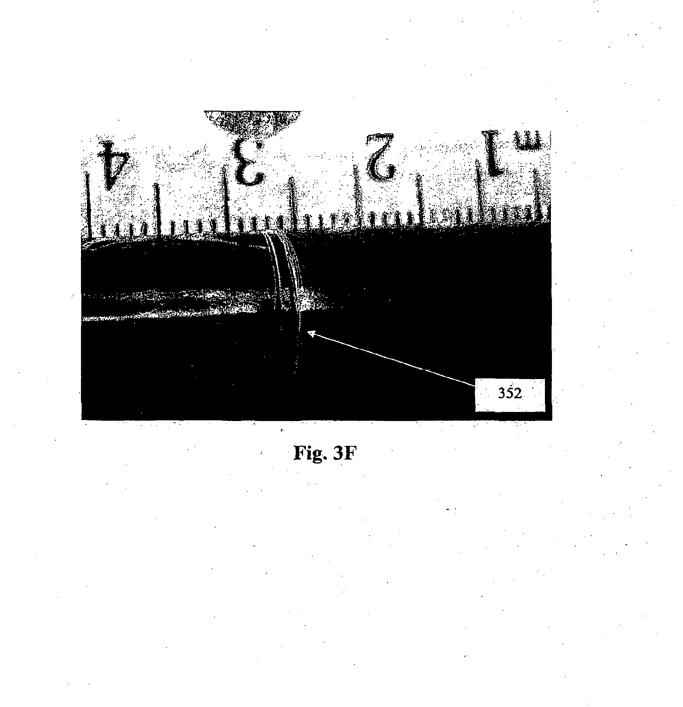

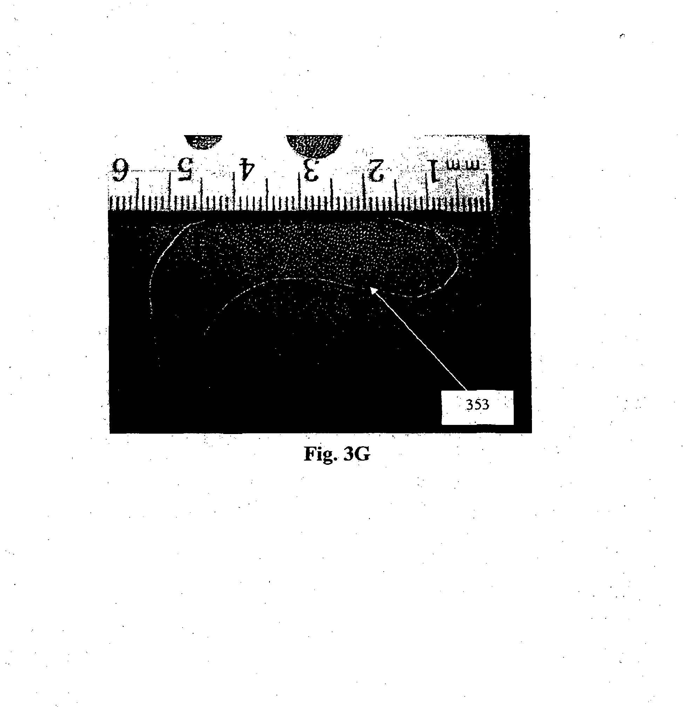

The strand, according to certain configurations, may have monofilament type structure, or multi-filament structure, and may appear like fine fishing line, sewing thread, yarn, or a suture. The photographs of Figures 3E-G are collagen product strands. The strand pictured in Figure 3E is a monofilament strand 351 similar to a fishing line. Figure 3F is another photograph of collagen product strands 352 wrapped around a spindle. Figure 3G is another photograph of a towed and twisted collagen product strand. As compared to the collagen product strand in Figure 3F, the collagen product strand 353 in Figure 3G is more robust and appears yarn-like. Each of the strands pictured may be prepared by wet extruding through a spinneret (single and multi-ported) resulting in a multitude of collagen product fibers being assembled in a linear agglomeration while being cross-linked, precipitated, and dewatered. The strand may be about 50 nanometers to about 3 millimeters, or about 50 microns to about 200 microns in diameter. The strands may be a fiber mass of loosely formed fibers that cling together by crimping or by their surface geometry, similar to how cotton fibers cling together. The strands may be slightly twisted or spun to form a strand having a more uniform diameter resembling sewing thread. In addition, the strands may be formed into a ribbon by positioning multiple strands side-by-side and drying the strands. In another example, the ribbon may be twisted to form a collagen product strand similar to a sewing thread, which may or may not be thicker than the twisted strand of collagen product described above. For example, a collagen product rope may be formed using the collagen product strand, which may have a diameter of between about 200 microns to about 3 millimeters. The strands resembling sewing thread may be about 100 microns to about a millimeter or more (e.g., about 2 mm to about 5 cm) in diameter. Collagen product strands may be used in further processes including weaving, knitting and/or braiding, for example. Alternatively, the stand may be formed by electrostatic spinning in which high electrical energy is used to form a Taylor Cone and send fiber bursts to a ground plate for deposition. The fiber begins in a collagen product dispersion that exits the electrostatic cone in a liquid form and is dried into a fiber during its flight to a grounding plate. The resulting fiber may be about 50 to about 400 nanometers in diameter. Each of the above-described collagen product strands may be prepared for use as a medical implant or may be further processed into, for example, a collagen product patch, in accordance with certain embodiments of the present invention.

When the collagen thread formed from the intermediate collagen product is used to produce medical implants such as a repair patch or sling (Figures 7 and 8), a pliable sheet of collagen product may result that may be sutured around the area to be repaired. For example, a non-woven repair patch (Figure 7) may be formed using a collagen product thread by employing a felting process. In addition, a repair patch 801 or sling may be woven, braided, and/or knitted (Figure 8), or may be formed from a combination of two or more of weaving, braiding (flat, three-dimensional, etc.) and knitting. Additional tissue repair fabrics and tissue repair fabric production methods are described in U.S. Patent No. 5,733,337, issued on March 31, 1998, entitled "Tissue Repair Fabric." In a further alternative, the collagen product sheet may be formed by any of the above-mentioned processes and formed into tubes, e.g., tubes 1301 from Figure 13, for applications such as vascular and neural repair, described further below.

Repair patches may be useful in applications such as: hernia repair, spinal tension band, annular repair for the spine, and/or for repair, reconstruction, augmentation or replacement of a sphincter, meniscus, nucleus, rotator cuff, breast, bladder, and/or vaginal wall. Accordingly, the repair patch or sling may be used in general surgical settings, in spinal, vascular, and/or neurosurgical settings, and/or for sports medicine surgical applications.

### Injectable Collagen Products

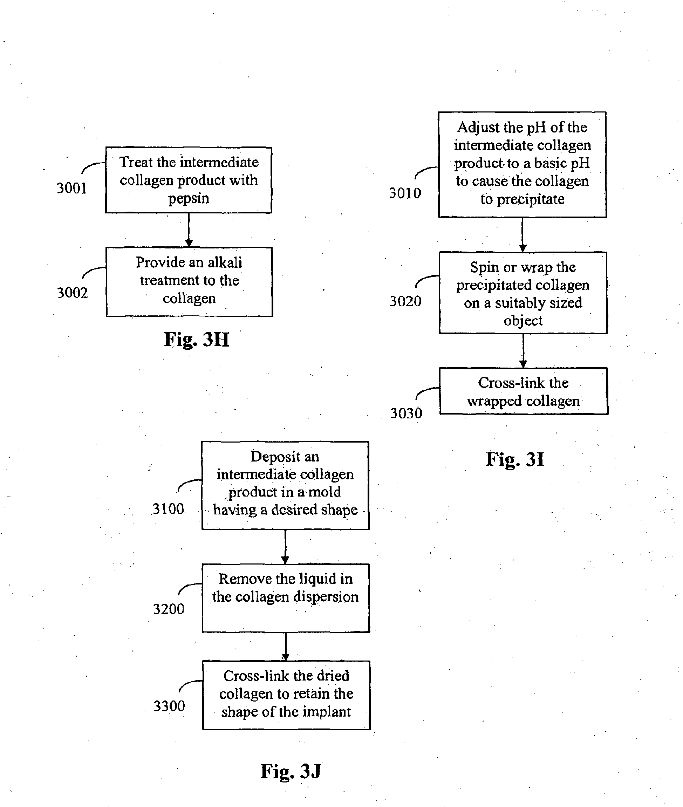

The intermediate collagen products may be use to produce an injectable form of collagen. According to Figure 3H, the intermediate collagen product may be treated (3001) with pepsin to remove telopeptides, and subjected to an alkali treatment (3002) so that, when implanted, the collagen product produces no or a low inflammatory response. The injectable collagen product may be useful in applications such as: scar revision, contracture revision, hypertrophic scar treatment, cosmetics, cosmetic surgery, wrinkle removal, cell delivery, drug delivery, clear collagens, dispersed collagens, micronized collagens (cryogenic grinding), and/or collagen product mixtures, e.g., collagen mixed with thrombin. Accordingly, injectable collagen products may be useful in various medical fields including plastic surgery, dermatology, and/or amputee stump revision.

Some methods that use non-human fascia to prepare soft tissue filler, which may be useful in accordance with some embodiments of the present invention, are described in U.S. Patent Application Publication No. 2002/0016637, published on February 7, 2002, entitled "Soft Tissue Filler;" and in Steven Burres, MD, Preserved Particulate Fascia Lata for Injection: A New Alternative, 25 Dermatologic Surg., 790-794 (October 1999).

### Collagen Product Tubes

The intermediate collagen product may be processed and formed into tubes for use as vascular (Figure 13) and/or neural grafts. Various processing techniques may be employed to construct a tube-like structure that may serve as vascular material or as a stent. According to the method of Figure 3I, vascular/neural graft is made by adjusting (3010) the pH of the intermediate collagen product to a more basic condition, resulting in the collagen product fibers and/or threads partly or fully precipitating. The precipitated collagen product fibers and/or threads may be firm and entangled, while being at least partly suspended in the water media, and may be easily be spun or wrapped (3020) onto a dowel or mandrel of a size suitable for reproducing the vascular/neural tissue to be repaired. The resulting grafts may be cross-linked (3030) to maintain their shape after removal of the dowel. In addition to fabricating vascular and neural grafts using the process described above, other implants that may be fabricated include: maxillary reconstruction tubes, which also contain mineral or allograft material, and/or hernia repair implants. Collagen product tubes may accordingly be useful in craniomaxillofacial, vascular, neurological, and/or general surgical applications.

### Collagen Product Plugs

Other medical implants such as plugs, meniscus repair structures, or cartilage repair structures 901 (Figure 9) may be formed using the intermediate collagen product of the present invention. For example, in the method of Figure 3J, an implant structure is formed by depositing (3100) a collagen product dispersion in a mold having a desired shape, removing (3200) the liquid in the collagen product dispersion, for example by lyophilizing, and cross-linking (3300) the implant in order to retain its desired shape. For an implantable plug, the dispersion may be deposited into a bullet-like mold, for example. Liquid may be removed from the dispersion using any suitable method including by lyophilization. Subsequently, the lyophilized collagen product structure may be cross-linked so that the implant retains its shape. In another example, the collagen product dispersion is mixed with a suitable biocompatible substance before depositing the dispersion into the mold. Collagen product plugs may be useful in cardiovascular surgical applications where the plugs may be inserted into vasculature to treat certain conditions, such as "blue baby" conditions. Collagen product plugs may be compressed by, for example, twisting, so that they can be inserted into the surgical site through a catheter. Upon rehydration in the surgical site, the plugs will assume their original shape.

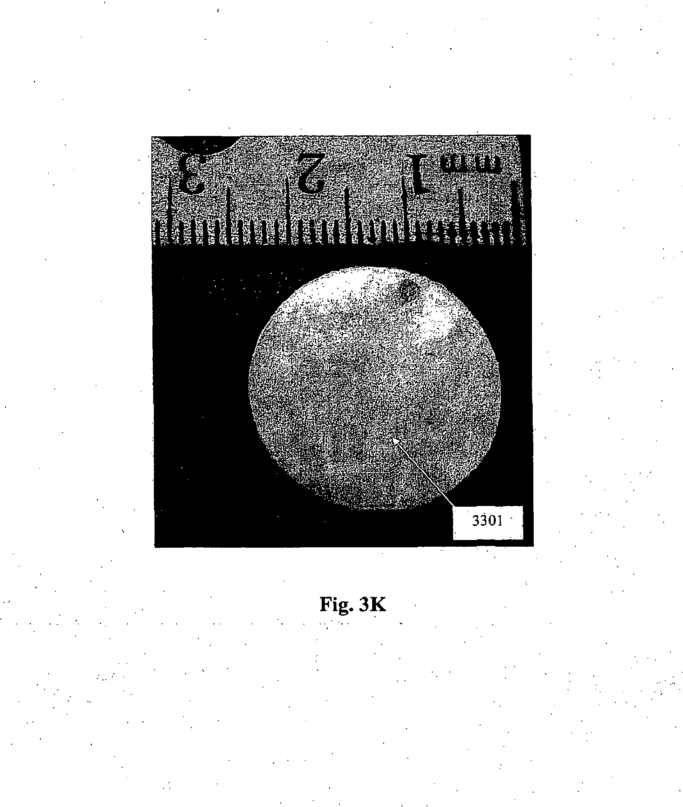

A collagen product plug 3301 is depicted in the photograph of Figure 3K, in which the collagen is processed in a similar manner compared to the human-derived collagen product scaffolds described below except the collagen product is bounded by a form or a mold. From Figure 3K, the collagen product plug 3301 has about a 22 mm diameter. However, the collagen plug may be of any suitable diameter depending on its intended use.

### In vitro Collagen Product Applications

The intermediate collagen product may be used in any suitable context. For example, the intermediate collagen product may be useful for *in vitro* applications and may be prepared for *in vitro* applications by various methods. For example, collagen products may be precipitated by any suitable method. Alternatively, the intermediate collagen product may be preserved, e.g., Figure 1A, and may be used for *in vitro* applications. The intermediate collagen product or the precipitated collagen product may be useful in applications such as for the manufacture of *ex vivo* tissue engineered products, cell culture media, and/or assays. Accordingly, collagen products for *in vitro* applications may be used in the cell tissue and engineering industry and/or in the medical testing industry.

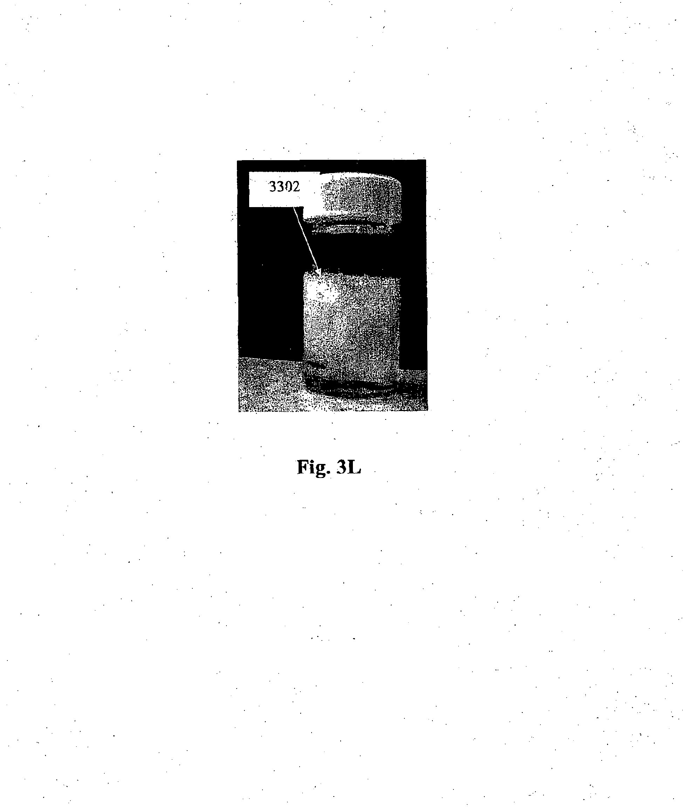

Figure 3L is a photograph of precipitated collagen product fibers 3302 in a vial having been precipitated from a collagen product dispersion. The precipitated collagen product may be of a self-assembling type where, once a precipitating agent is added to the collagen product suspension, collagen product fibers precipitate into the solution that appear like a broken-apart cotton ball. Such an en masse precipitated collagen product is easily recovered from the solution and may be used as a medical implant, or may be further processed depending on the *in vitro* application of the human-derived collagen product.

### Collagen Product Scaffolds

### Collagen Product Scaffolds Formed from Intermediate Collagen

### Product I

Intermediate collagen product I may be formed into a collagen scaffold according to the method described in Figure 2D in U.S. Patent Application No. 11/673,972, filed February 12, 2007, entitled "Methods for Collagen Processing and Products using Processed Collagen."

### Collagen Product Scaffolds Formed from Intermediate Collagen Products II-III

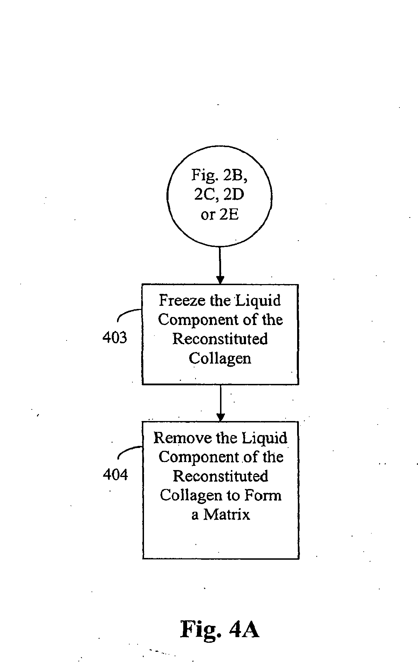

Alternatively, intermediate collagen product II or III may be forked into a collagen product scaffold according to the methods described below. According to Figure 4A, an intermediate collagen product produced according to Figure 2B, 2C, 2D or 2E is frozen (401) and the liquid component is removed (402) to yield a medical implant.

According to the invention, an alcohol, such as EtOH, or another substance, which forms the intermediate collagen product II or III, remains in the dispersion when the mixture is frozen and causes the freezing characteristics of the intermediate product to be altered. For example, the crystal size of the ice crystals in the frozen intermediate product containing alcohol may be controlled. Other agents also may be used to control ice crystal formation and size. As a result, the quality of the finished collagen product may be more accurately predicted and/or controlled because controlling crystal size allows the size of the void spaces, i.e., interstices, resulting from removal (such as lyophilization) of the water and alcohol component, and the fiber size of the collagen product to be controlled. Thus, collagen products may be produced that have void spaces similarly sized, e.g., a narrow size distribution of the void spaces, distributed evenly, e.g., homogenous, with a desired pore density, resists cracking, has a high degree of plasticity, and/or an end product that is stronger compared to collagen products not having alcohol in the dispersion. That is, freezing characteristics of collagen product dispersions where there is no agent controlling ice crystal size may result in uncontrolled ice crystal size during freezing, resulting in a wide range of void space sizes. As a result, large shard ice crystals may result in large and/or uneven void spaces in the finished products, which may cause weaknesses and/or brittleness in the finished product.

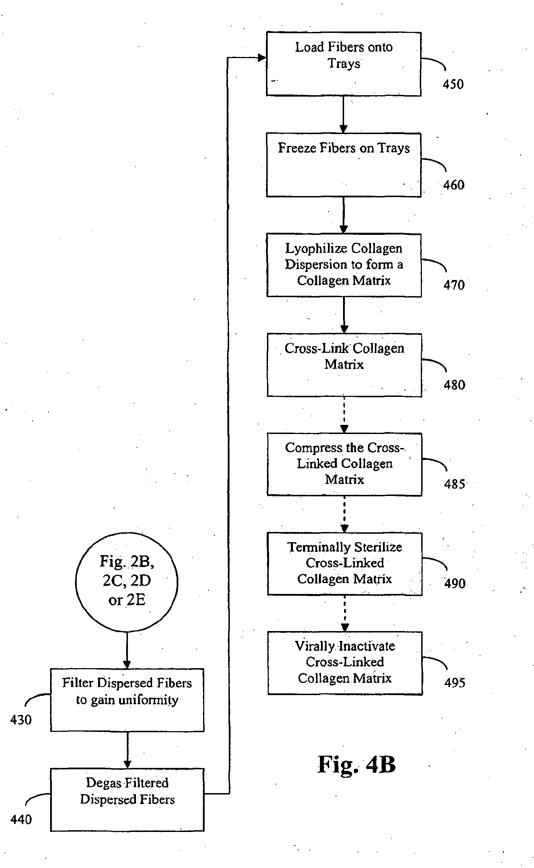

Another method for producing a collagen product scaffold provided in Figure 4B. According Figure 4B, a wound repair scaffold or matrix is produced by following the steps of Figure 2B, 2C, 2D or 2E. The intermediate collagen product in alcohol may be filtered (430), which may enhance uniformity. For example, the dispersion may be filtered through a woven screen mesh having 0.024" round or square openings, through a woven or perforated stainless steel screen having about 8 to 24 gauge holes, or through a screen having a series of openings that form about a 30% open area. Filtering may be repeated to ensure a uniform dispersion. In some embodiments, the filtering is conducted at a desired temperature, e.g., a temperature of ≤ about 15 °C, or any other desired temperature or range of temperatures.

The filtered collagen product may be subsequently degassed (440), which may affect the porosity of the finished product. In one example, the collagen product is degassed via centrifugation, e.g., at about ≤ 15 °C, which can eliminate large irregular pockets of gas or air. In addition or alternatively, the collagen product may be degassed by vacuuming. The degassed product may be collected by slow decant while discarding any precipitate, such as dense collagen particles resulting from lactic acid not penetrating interior collagen product fibers and/or threads in a dense fiber bundle or pellet.

The filtered collagen product is or can be loaded (450) into stainless steel or aluminum trays to a depth ranging from any thickness greater than 0 mm to several inches thick, or about 0.5 mm to about 35 mm, or at a depth of about 4 mm. For example, some dispersion depths may include about 5, 7, or 12 mm. However, the depth the collagen product is loaded into the trays is based on the desired end product thickness, which may be about 0.1 cm to about 15 cm in height, width, and depth, or about 12 cm to about 15 cm in height and width and about 0.1 mm to about 12 cm in depth, or any suitable dimension.

The trays loaded with the collagen product dispersion product may be frozen (460). For example, the trays may be frozen from room temperature, e.g., about 18-23 °C, to a temperature of about -20 °C to about -60 °C, or about -30 °C to about -50 °C, for a duration of about 6 hours, for example, to achieve a uniformly frozen dispersion. This may be accomplished in any suitable manner, including by freezing the product in a freezer or lyophilizer.

Once frozen, the collagen product dispersion may be lyophilized (470) to maintain the shape and distribution of the collagen product sponge matrix while removing the liquid, e.g., water and alcohol, components of the dispersion. According to certain embodiments, a lyophilizer is programmed to conduct a number of cycles, each cycle haying a set temperature, at a given vacuum pressure and for a given period of time. For example, the temperature inside the lyophilization chamber can be in the range of about -70 °C to about +30 °C, the vacuum pressure can range from about 90 Millitorr to about 2000 Millitorr, and the duration for each cycle may range from about 1 hour to about 10 hours. It will be understood that the cycle parameters may be selected and/or adjusted in order to remove the water component of the collagen product dispersion without causing the collagen product matrix to collapse or become damaged.

In some embodiments, the lyophilized collagen product matrix may be cross-linked (480) to maintain the matrix in a desired form, to impart desirable mechanical properties of the finished matrix, and/or to control the residence time of the matrix after implantation. In certain embodiments, cross-linking may be achieved by exposing the lyophilized collagen product matrix to a cross-linking agent. Chemical cross-linking agents include those that contain bifunctional or multifunctional reactive groups, and which react with functional groups on amino acids such as epsilon-amine functional group of lysine or hydroxy-lysine, or the carboxyl functional groups of aspartic and glutamic acids. By reacting with multiple functional groups on the same or different collagen molecules, the reacting chemical cross-linking agent forms a reinforcing cross-bridge. Cross-linking agents may include: monoaldehydes, dialdehydes, polyepoxy compounds, polyvalent metallic oxides, chemicals for esterification of carboxyl groups followed by reaction with hydrazide to form activated acyl azide functionalities in the collagen, organic tannins and other phenolic oxides derived from plants, tanning agents, glycerol polyglycidyl ethers, polyethylene glycol diglycidyl ethers, sugars, enzymes, and heterobifunctional crosslinking agents. Particular examples of vapor phase gasses may include: formaldehyde, glutaraldehyde, acetaldehyde, polyepoxy and diepoxy glycidyl ethers, titanium dioxide, chromium dioxide, aluminum dioxide, zirconium salt, glyoxal pyruvic aldehyde, dialdehyde starch, dicyclohexyl carbodiimide hydrazide, dicyclohexyl carbodiimide, hexamethylene diisocyanate, dicyclohexyl carbodiimide and its derivatives, hexamethylene diisocyanate, glucose, and genipin. Genipin is a naturally-occurring cross-linker, which is discussed in various articles including: Sung HW, Chang Y, Liang IL, Chang WH, Chen YC. Fixation of biological tissues with a naturally occurring crosslinking agent: fixation rate and effects of pH, temperature, and initial fixative concentration. J Biomed Mater Res 2000;52(1):77-87; Huang LL, Sung HW, Tsai CC, Huang DM. Biocompatibility study of a biological tissue fixed with a naturally occurring crosslinking reagent. J Biomed Mater Res 1998;42(4):568-76; Tsai CC, Huang RN, Sung HW, Liang HC. In vitro evaluation of the genotoxicity of a naturally occurring crosslinking agent (genipin) for biologic tissue fixation. J Biomed Mater Res 2000;52(1):58-65; Sung HW, Huang RN, Huang LL, Tsai CC, Chiu CT. Fcasibility study of a natural crosslinking reagent for biological tissue fixation. J Biomed Mater Res 1998;42(4):560-7. Glutaraldehyde cross-linked biomaterials have a tendency to over-calcify in the body. In this situation, should it be deemed necessary, calcification-controlling agents can be used with aldehyde crosslinking agents. These calcification-controlling agents include: dimethyl sulfoxide (DMSO), surfactants, diphosphonates, aminooleic acid, and metallic ions, for example ions of iron and aluminum. The concentrations of these calcification-controlling agents can be determined by routine experimentation by those skilled in the art.

In certain embodiments, cross-linking may be achieved by exposing the lyophilized collagen product matrix to a cross-linking agent in the form of a vapor phase gas including gasses of one or more of the above-listed cross-linking agents. Any suitable cross-linking method may be used. For example, the collagen product matrix may be suspended in a vessel holding a volume of aldehyde solution sufficient to cover the bottom of the vessel. The vessel with the matrix suspended inside may be covered for a suitable period of time, e.g., a range of about 15 minutes to 2 hours, to which allow the vapor phase of the aldehyde to cause vapor phase cross-linking at a suitable temperature, e.g., 18-23 °C. Alternatively, the lyophilized collagen product matrix may be cross-linked by dehydrothermal cross-linking, by subjecting the matrix to ultraviolet light, or by any other suitable method. Various cross-linking methods and cross-linking agents are described in U.S. Patent No. 6,123,731, issued on September 26, 2000, entitled "Osteoimplant and Method for its Manufacture."

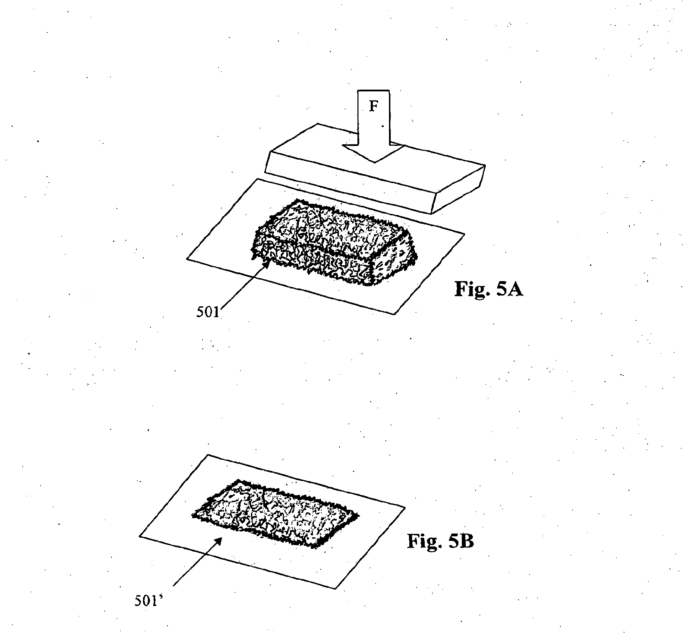

According to certain implementations, the cross-linked collagen product may optionally be compressed (485) to yield a collagen product with a smaller thickness compared to its pre-compression thickness. For example, the compressed product may be 2/3, 1/2, 1/3, 1/4, 1/10, 1/20, 1/30, 1/40, or 1/50 to 1/100 the thickness of the original product thickness. In a particular example, for a 4 mm collagen product sponge, compressing at about 125 to 175 psi, about 150 psi, or about 6,000 pounds force on a 4"x5" collagen product, for about 30 seconds yields a collagen product with a thickness of about 0.13 mm. The compressed product may resemble a pliable sheet or film having a paper-like appearance. Figure 5B depicts a collagen product sheet formed as a result of compressing the collagen product sponge in Figure 5A by force "F." Furthermore, in some embodiments, the cross-linked collagen product may be cut to size, molded to size, or embossed in addition to or as an alternative to being compressed. In alternative embodiments, the matrix may be compressed and subsequently cross-linked, which may provide a matrix that has a smaller thickness compared to a matrix that is cross-linked and compressed.

In some embodiments, the cross-linked matrix may be terminally sterilized (490) and/or virally inactivated (495). Any suitable terminal sterilization method may be used, including ethylene oxide gas treatment, cobalt radiation, gamma irradiation, electron beam radiation, gas plasma processing, etc. Sterilization and/or viral inactivation methods are provided in Brown P., et al., Sodium hydroxide decontamination of Creutzfeld-Jakob Disease virus, New England J. of Med. Vol. 310, No.11; Abe S., et al., Clinical experiences with solvent dehydrated fascia lata in plastic surgery, Jap. J. Plast. Reconst. Surg. 1991, Vol. 11, 721-730; and Hinton R., Jinnah R.H., Johnson C., et al., A biomechanical analysis of solvent-dehydrated and freeze-dried human fascia lata allografts, Am. J. Sports Med. 1992, 20: 607-612.

In addition to or as an alternative to sterilizing, the cross-linked collagen product matrix may be packaged for subsequent use as a wound repair matrix. Packaging the collagen product may protect it from environmental conditions. When the collagen product is compressed, the product may be sealed in an envelope or plastic bag. The compressed product may be prepared for use by, for example, wetting the compressed sheet-like material. In some embodiments, wetting may cause the collagen product to return to its original sponge-like state, for example, when compression occurs after cross-linking. Alternatively, wetting may cause the collagen product to expand to a shape smaller than its original sponge-like state, for example, when compression occurs before cross-linking. The wetting process may include immersing the collagen product in water or spraying the collagen product with water or saline, e.g., about 0.9% saline, and may take place in a medical setting such as an operating room. Alternatively, when the collagen product is not compressed and resembles a sponge, the product may be sealed in a tray and used in medical settings.

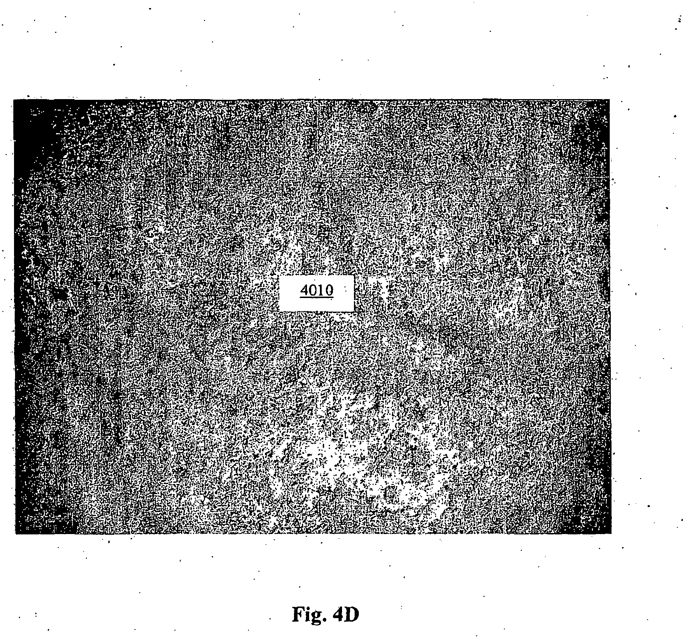

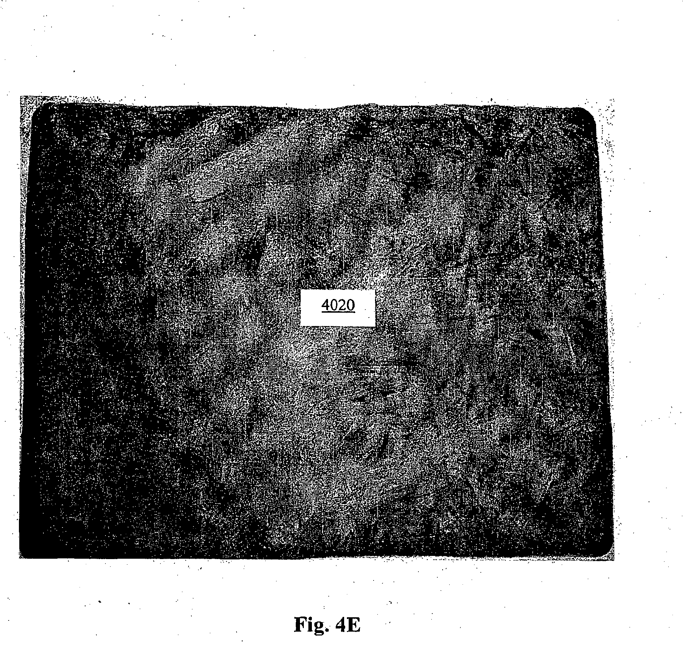

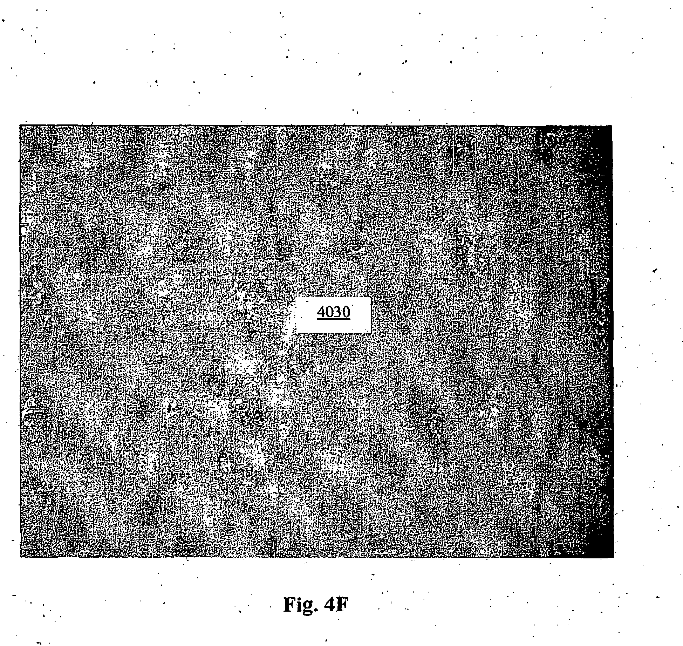

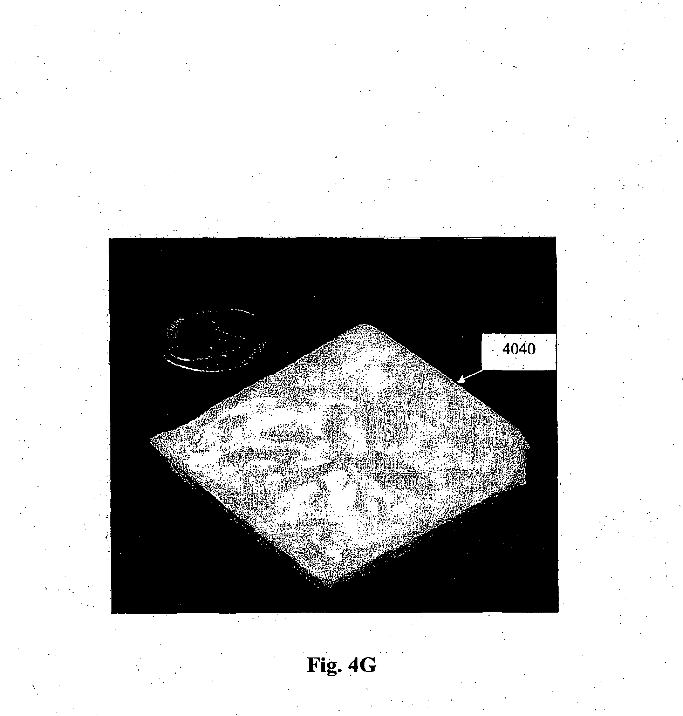

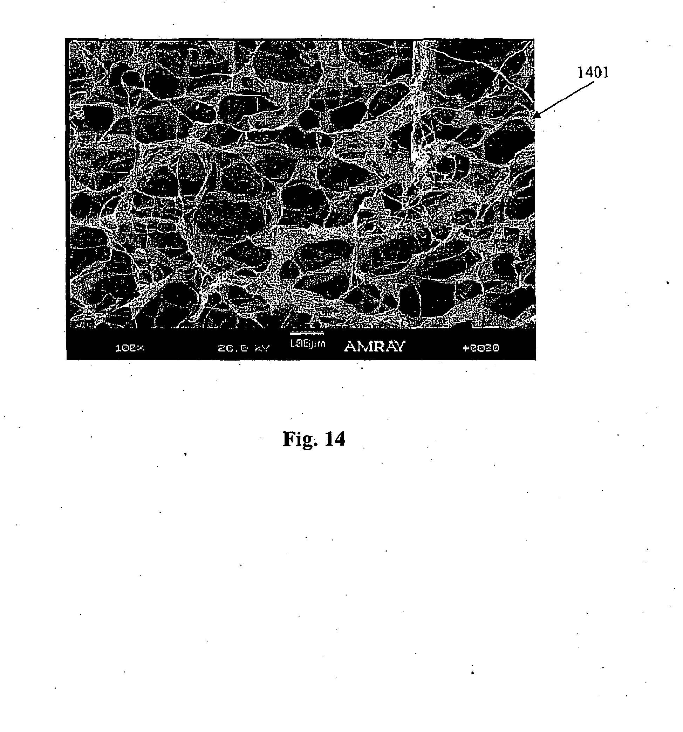

Wound repair scaffolds produced according to the methods of Figures 4A and 4B, U.S. Patent Application No. 11/673,972, and variants thereof, are depicted in Figures 4D-G, Figures 5A-B, Figure 6 (Note, the scaffold of Figure 5A is the same as the scaffold 601 of Figure 6), and Figure 14, in which the wound repair scaffold resembles a collagen product sponge or film. The photographs of Figures 4D-G depicts collagen product scaffolds 4010, 4020, 4030 and 4040 that are between about 3 to about 6 mm thick that are made from human fascia in the form of an intermediate collagen product containing 5% ethanol that has been frozen, lyophilized and cross-linked with a suitable cross-liking agent for about an hour. The resulting human-derived collagen product 4010 in Figure 4D is characterized by crystal patterns having a narrow size distribution. In Figure 4E, the resulting human-derived collagen product 4020 is characterized by a small amount of crystal sharding on the top right side of the sample, and a near homogenous or uniform scaffold product on the bottom left side with no crystal sharding. The photographs of Figure 4F and 4G each show.a collagen product matrix 4030, 4040 having a marbling pattern across the top surface. A marbled appearance in the end product is desirable because the appearance of an opaque white foam is evidence of the absence of an ice crystal pattern, an amorphous surface, and void spaces with a narrow size distribution. The collagen products 4030, 4040 pictured in Figures 4D-G are acceptable collagen products for use as a medical implant because the collagen products do not include quality deviations, e.g. large crystal shard borders, that may cause cracking. This is due to the presence of alcohol in the frozen dispersion, because by its presence the crystal nucleation and crystal size may be controlled during freezing resulting in no or small crystals that are bounded. In contrast, when a collagen product does not include alcohol in the freezing and/or lyophilizing steps in the production method, a thin, nearly transparent foam with frost-like patterns similar to the appearance of onion skin results, which is brittle and prone to cracking when stressed. While the above embodiments are between about 3 mm and about 6 mm thick, it will be understood that collagen product scaffolds produced according to the invention may be between about 1 mm and about 12 mm thick, between about 3 mm and about 6 mm thick, or about 3.5 mm thick.

A collagen product scaffold 1401 made from human fascia is depicted in Figure 14, which is a photograph, taken at 100x magnification by scanning electron microscopy. The collagen product scaffold may be prepared according to the scaffold production methods described above, and may be used as a medical implant in accordance with certain embodiments of the present invention. The human-derived collagen product in Figure 14 results from crystal patterns having a narrow size distribution, which results in a collagen product having a desirable distribution of pore size and pore density.

The sponge-like scaffold 501 of Figure 5A results from collagen product production methods that do not include a compression step. In contrast, the film-like scaffold 501' of Figure 5B results from collagen product production methods that do include a compression step.

It will be noted that Figure 5B depicts a wound repair collagen product scaffold 501' in the form of a sheet or film that may be produced in accordance with the methods of Figure 4A or 4B combined with a compression step. Compressing the collagen product after lyophilizing and cross-linking results in a flexible compressed collagen product. Compressing the collagen product after lyophilizing but before cross-linking results in a slightly less flexible compressed collagen product but with stronger resistance to suture tear-out upon rewetting. Both compressed collagen products are in contrast to a bovine collagen scaffold, which is comparatively stiff or board-like.

When the collagen product sheet or film is to be used as an implant, it is removed from its packaging, if present, and wetted, e.g., by wetting in saline, e.g., about 0.9% saline, so that the film or sheet expands into its original sponge-like shape, e.g., into the collagen product scaffold depicted in Figure 5A, or into a sponge-like shape that may be thinner compared to its original pre-compressed shape when the collagen product scaffold is compressed before, and in some embodiments after, cross-linking. The rewetted collagen product implant is ready for implantation and may be any or all of flexible, drapeable, capable of forming a seal with adjacent structures, strong and/or resistant to suture pullout. In addition, a pressed collagen product, provided according to certain embodiments, retains a pressed flat condition, whereas a bovine collagen scaffold does not.

A drapeable scaffold, e.g., a scaffold that is prepared by relofting, has improved capability to conform to an implant site, e.g., the brain. A scaffold resistant to suture pullout or tear-out provides a collagen product implant that demonstates improved ability to be sutured without buckling or lifting. In some implementations, where relofting results in an implant that slightly thinner than the original thickness, the collagen product exhibits improved strength and is more resistant to suture pullout or tear-out. A drapeable and suturable scaffold can provide a gentle yet comprehensive seal at an implant site.

The sponge-like or film-like wound repair scaffolds of Figures 4D-4G and 5A-B may have various applications and may be used as a dura/meningeal repair dressing, sponge-like or foam-like or otherwise absorbent hemostat, dermal repair dressing, cartilage repair scaffold, cell growth media, and/or substance delivery media, e.g., drugs, nutrients, growth factors, etc. Wound repair matrices may be used in combination with other medical implant structures with or without human-derived or human-like collagen components. Matrices fabricated according to certain implementations may be flexible, tough, soft, drape-like, have a high degree of plasticity, and/or be resistant to suture pullout; and may be useful in applications such as neurosurgery, orthopaedic surgery, laboratory applications, dermatology, and/or plastic surgery.

### Example: Comparison of Collagen Scaffolds based on Starting Collagen Material

Scaffold characteristics maybe at least partly dependent on the source of the collagen used to prepare the intermediate collagen product. For purposes of example, bovine tendon is compared to human tendon. A dispersion of about 0.75% bovine collagen derived from bovine tendon is more viscous, e.g., has a thickness of honey, and results in a stiffer sponge compared to a dispersion of about 0.75% human collagen derived from human tendon, which is comparatively thinner (e.g., slightly more viscous than water) and is self-leveling. In another example, bovine fascia is compared to human fascia. A dispersion of about 0.75% bovine collagen derived from bovine fascia is a non-free-flowing highly viscous dispersion that results in a stiffer sponge compared to a dispersion of about 0.75% human collagen product derived from human fascia, which on the other hand, is free-flowing (e.g., nearly water-like) and self-leveling. Each of the human-derived products has a beige color and their dispersions may have a yellow/green color, whereas bovine dispersions and products are relatively white. The resulting human-derived collagen product scaffold has a higher degree of plasticity and elasticity compared to the bovine sponge, which allows the scaffold to generally return to its original shape when manipulated. Further, human-derived collagen product scaffold is flexible and resistant to cracking which allows the scaffold to be bent and twisted without creasing. In addition, the scaffold made from the human collagen product has better draping and handling properties, which allows the scaffold to be wetted and conformed and adhered to an implant area. Moreover, for human-derived collagen products made from tendon compared to fascia, a tendon-sourced collagen product scaffold is stiffer compared to the fascia-sourced collagen product scaffold, but both are more elastic and plastic compared to bovine-derived collagen scaffolds. Although, human-derived fascia and tendon are contemplated as a starting material for producing collagen product scaffolds, intermediate collagen products and other collagen implants may be produced using other human-derived sources, e.g., any type of human-derived collagen. It will be appreciated, however, that bovine-sourced collagen-containing tissue and other non-human collagen-containing tissue may be used as a starting material according to certain aspects of the invention, and the above comparison should not be construed to mean that bovine or non-human-sourced collagen is unsuitable for embodiments of the invention. For example, non-human tissue may be enzymatically treated to remove immunilogically active gylcoproteins and recombinant collagen, while retaining non-collagenous proteins that may provide beneficial effects in humans. Accordingly, non-human derived tissue may be processed in a similar or same manner as the methods described above in order to provide an implant that produces no or a low immunogenic response in humans.

### Example: Comparison of Collagen Product Scaffolds based on Intermediate Collagen Product

Collagen product scaffolds have different physical characteristics when formed from intermediate collagen product II, III, and an intermediate collagen product that contains the liquid component of the blended collagen product dispersion, i.e., with any foam removed.

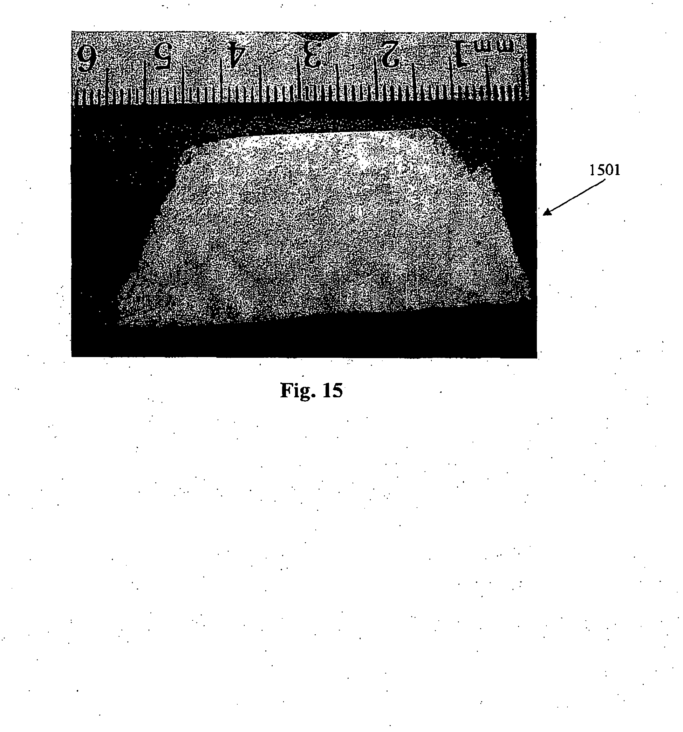

A collagen product scaffold made from intermediate collagen product II, .e.g. a reconstituted collagen product foam layer from human-derived fascia and a leveling agent, is substantial, resists deformation and tearing when handled roughly, but is pliable. Figure 15 is a photograph of a collagen product scaffold 1501 produced from intermediate collagen product II made from human fascia as a starting material, which may be prepared for use as a medical implant in accordance with certain embodiments of the present invention.

A scaffold produced from intermediate collagen product III, .e.g., collagen product from human-derived fascia and a leveling agent, is flexible, firm and has elastic/plastic characteristics that are substantially similar in both the x and y directions. However, the scaffold produced from intermediate collagen product III is not as substantive or strong compared to the scaffold produced from intermediate collagen product II.

A collagen product scaffold produced from a collagen product dispersion without a foam component, e.g., with the foam layer removed, is soft, sensitive to the touch, and easily deformable, not elastic, and tears upon rough handling. Accordingly, the collagen scaffolds formed from the collagen product intermediate II and III exhibits differing physical and mechanical properties.

### Characterization of Collagen Scaffolds

Collagen product scaffolds produced with intermediate product III, e.g., with the collagen dispersion and re-liquefied foam component, and subjected to various tests for characterization.

**Tensile Test:** Dry collagen product scaffolds formed from human fascia were cut into 12 mm x 80 mm samples and rehydrated in 0.9% saline solution for 5 minutes or at least until hydrated prior to testing. Samples were subjected to testing on a MTS® machine at a strain rate of 60 mm/min. The ultimate tensile strength for five samples, along with the average ultimate tensile strength and standard deviation are provided in Table 1.

**Table 1**

| Sample | Ult Stress (MPa) |
|---|---|
| A | 5.951 |
| B | 1.708 |
| C | 2.544 |
| D | 2.208 |
| E | 1.496 |
| Average | 2.782 |
| Std. Dev. | 1.819 |

**Suture Retention Test:** Collagen product scaffolds described above were cut into 10 mm x 20 mm samples and rehydrated. A 4-0 Ethicon silk thread in a tf-1 tapered needle formed a suture, 3 mm suture bite 20 mm width. A MTS® machine was run at a strain rate of 20 mm/min. The suture strength for five samples, along with the average strength and standard deviation are provided in Table 2.

**Table 2**

| Sample | Strength (N) |
|---|---|
| A | 0.582 |
| B | 0.651 |
| C | 0.546 |
| D | 0.582 |
| E | 0.624 |
| Average | 0.597 |
| Std. Dev. | 0.041 |

**Burst Strength Test:** Collagen product scaffolds described above were cut into 100 mm x 100 mm samples and rehydrated. A Mullen Burst apparatus was attached to a MTS® machine and a constant strain rate of 305 mm/min. was applied (ASTM 3787). The burst strength for five samples, along with the average burst strength and standard deviation are provided in Table 3.

**Table 3**

| Set | Burst (N) | @ Displacement (mm) | Linear Stiffness (N/mm) |
|---|---|---|---|
| A | 20.7130 | 13.9778 | 2.1687 |
| | 31.1333 | 16.1899 | 3.0481 |
| | 30.7907 | 13.9891 | 3.8112 |
| | 25.9281 | 13.1448 | 3.3830 |
| | 25.9233 | 15.1670 | 3.2199 |
| B | 34.1159 | 16.1587 | 3.2221 |
| | 27.4880 | 15.1560 | 2:6919 |
| | 22.1082 | 14.1627 | 2.2377 |
| | 37.4927 | 16.1831 | 3.8224 |
| | 39.6557 | 15.6670 | 4.0538 |
| C | 32.7945 | 15.3310 | 3.8650 |
| | 25:2914 | 13.9613 | 3.2255 |
| | 14.1775 | 12.1089 | 1.5695 |
| | 25.1739 | 14.8091 | 2.9499 |
| | 21.5813 | 14.6381 | 2.2134 |
| D | 18.8626 | 13.2826 | 2.4716 |
| | 32.4219 | 16.1484 | 3.2478 |
| | 20.4316 | 14.2975 | 2.0227 |
| | 25.1739 | 14.9851 | 2.6338 |
| | 26.8084 | 13.6325 | 3.1078 |
| Average Std. Dev. | 26.9033 | 14.6495 | 2.9483 |
| | 6.4641 | 1.1414 | 0.6849 |

**Denaturation Temperature Test:** Collagen product scaffolds described above were cut into 4 mm x 4 mm samples and rehydrated for 15 minutes. The samples were placed in an aluminum crucible, sealed and run in a DSC analysis at a temperature increase of 10 °C/min. The temperature a which each of the nine samples denatured, along with the average temperature and standard deviation are listed in Table 4.

**Table 4**

| Sample | Temperature (°C) |
|---|---|
| A | 63:0 |
| B | 62.3 |
| C | 61.5 |
| D | 56.7 |
| E | 56.8 |
| F | 58.2 |
| G | 61.0 |
| H | 56.8 |
| I | 56.8 |
| J | 58.9 |
| K | 56.7 |
| L | 58.1 |
| Avg. ± Std. | 58.9±2.4 |

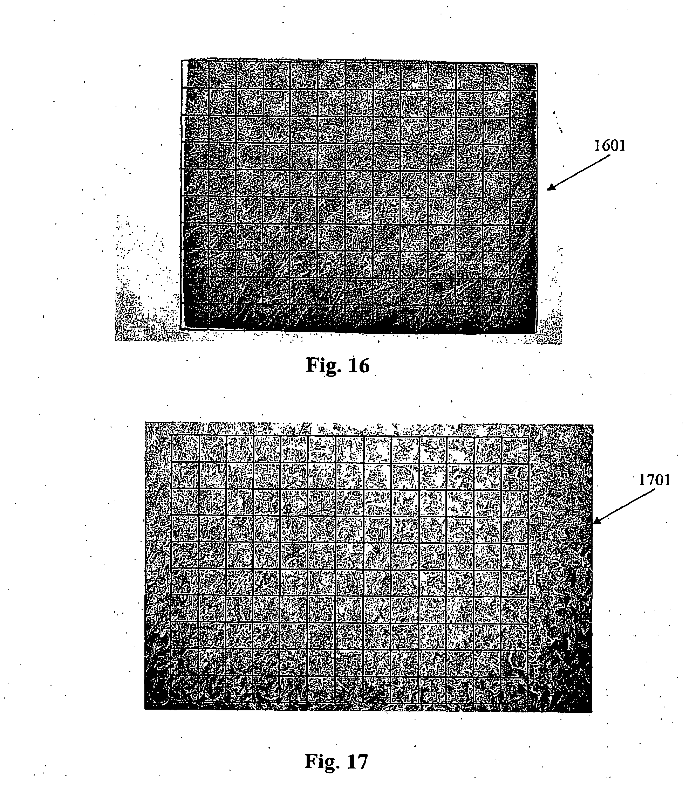

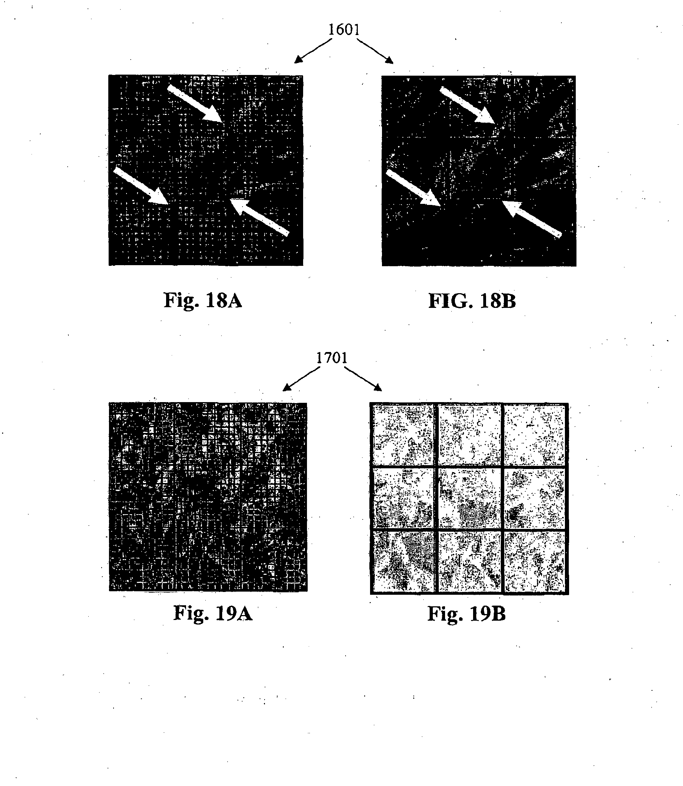

**Visual Characterization:** Lyophilized collagen product scaffolds produced according to certain embodiments of the present invention were compared to other collagen product scaffolds using stereology. Figure 16 is a photograph with grid overlay of a 13 cm x 10 cm collagen product scaffold produced according to known methods. The typical ice sharding pattern viewable in the collagen product scaffold 1601 of Figure 16 produces large shards spanning areas over several square centimeters. Figure 17 is a photograph of a collagen product scaffold 1701 with grid overlay of a 15 cm x 11 cm collagen product scaffold produced according to embodiments of the present invention. According to Figure 17, the ice sharding patterns are comparatively small. Figures 18A-B are 3 cm x 3 cm areas of the scaffold 1601 of Figure 16 showing an example large shard outlined by 3 arrows, which spans an area that is approximately 70 mm². Figure 18B provides a clear image of the large shard with the small grid lines removed. In comparison, Figures 19A-B are 3 cm x 3 cm areas of the scaffold 1701 of Figure 17 where each shard is approximately 11 mm². Figure 19B provides a clear image of the small shards with the small grid lines removed. In view of Figures 16-19, lyophilized collagen product scaffolds 1701 produced according to some implementations of the present invention are characterized by small shards when compared to lyophilized collagen product scaffolds 1601 produced by other means. It is believed that small shard patterning provides a stronger, more durable collagen product scaffold and that large shard patterning results in a weaker, less durable collagen product scaffold. Accordingly, collagen products, in particular lyopilized scaffolds, produced according to implementations of the present invention are high strength, durable and biologically compatible collagen product implants. In some implementations (not shown), collagen product scaffolds may be produced according to embodiments of the invention that further include the addition of glycerol to the collagen product suspension, which may affect the crystal size of the finished scaffold.

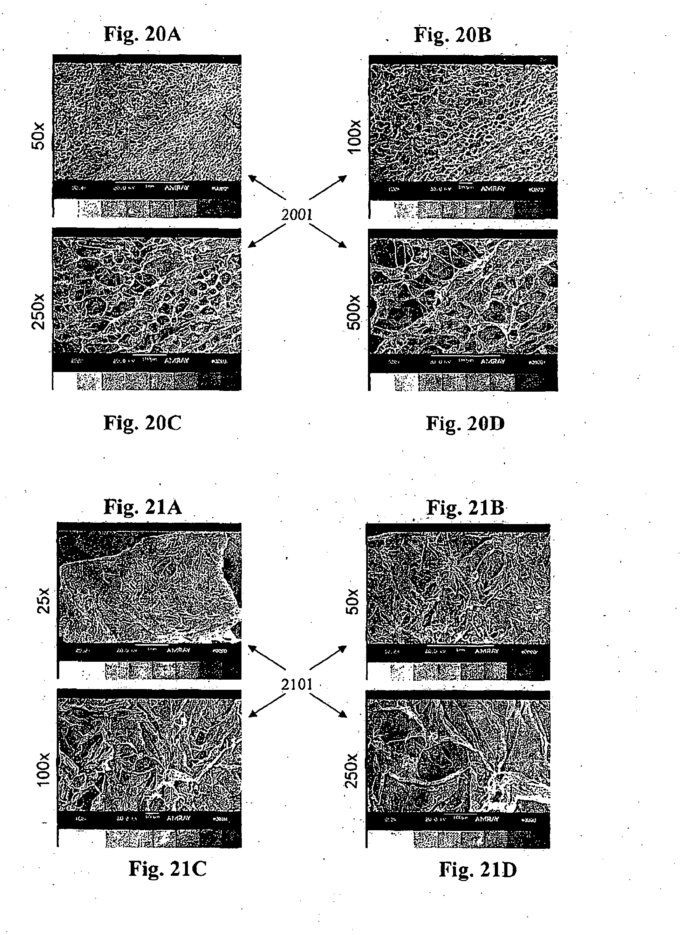

**Scanning Electron Microscope (SEM) Characterization:** Lyophilized collagen product scaffolds produced according to certain embodiments were compressed at 3000 lbs, reconstituted and dried. SEM images of the lyophilized scaffolds 2001 show pore structure on a first surface of the scaffold, e.g., top surface, at a magnification of 50x (Figure 20A), 100x (Figure 20B), 250x (Figure 20C) and 500x (Figure 20D). SEM images of another lyophilized collagen product scaffold 2101 shows the scaffold structure of another surface of the scaffold, e.g., bottom surface or the scaffold surface that is directly adjacent to the surface it was frozen upon, at a magnification of 25x (Figure 21A), 50x (Figure 21B), 100x (Figure 21C) and 250x (Figure 21D). From Figures 20A-D, the pore structure of the collagen product scaffold 2001 is relatively uniform. Uniformity in pore structure may provide a substantial and pliable implant that resists deformation and tearing when handled roughly.

### Collagen Product Matrix/Sling

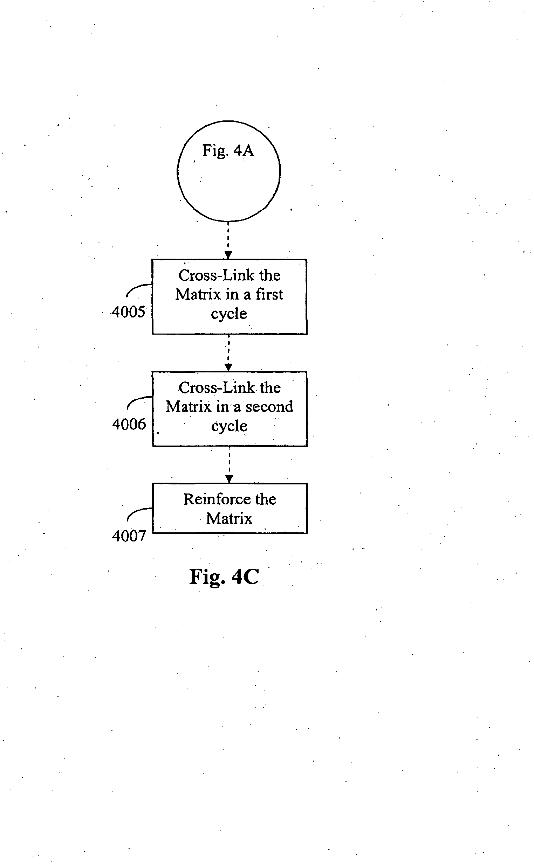

In further embodiments, the matrix or scaffold produced according to the methods depicted in Figures 4A, 4B, U.S. Patent Application No. 11/673,972, and variants thereof, may be further processed to alter or add material to the scaffold. For example, according to Figure 4C, the liquid component is removed to form a matrix from Figure 4A, and one or more cross-linking cycles (4005, 4006) may be added to the production process that will increase the density of the scaffold.

In addition or alternatively, and according to Figure 4C, the scaffold may be reinforced (4007) by adding to the scaffold PEEK film, polylactide and polypropylene sutures, bone, metal implants (e.g., steel), any number of bio active polymers, e.g., tyrosine polycarbonates and tyrosine polyarylates, bio active drugs in poly form, and/or other biocompatible materials. Processes for adding reinforcing materials to the matrix may include: lamination, vapor deposition, dispersion, and/or chemical reaction. In addition, the altered collagen product matrix or scaffold may be compressed. Moreover, collagen products may be altered by providing one or more of the above-mentioned materials inside of the collagen product matrix. For example, PEEK film may be provided as a mesh or other reinforcing component and the collagen product matrix may be formed over and around the mesh.

Altered collagen product matrices described above may be used as a repair matrix or sling in applications such as rotator cuff repair, breast reconstruction or augmentation, hernia repair, vaginal wall repair, sphincter repair, meniscus repair, and/or annular repair of the spine. Accordingly, the altered collagen product may be useful in general, orthopaedic, obstetric, gynecological, plastic, and/or urological surgical settings, for example.

Although the intermediate collagen products I-III may be produced according to the above-described methods in which isolated collagen fibers and/or threads are used to produce the intermediate collagen product, e.g., previously recovered dried and dehydrated collagen, intermediate collagen products I-III produced during the collagen recovery process are also contemplated. For example, collagen recovery methods including the methods described in the above-mentioned Patent Application No. 11/673,972, filed February 12, 2007, entitled "Methods for Collagen Processing and Products using Processed Collagen," may include a processing step in which a leveling agent, e.g., alcohol or a salt, is blended with the collagen so that the collagen is suspended in a foam and liquid layer. The foam may be recovered and the collagen product therein further processed according various collagen recovery steps in order to prepare collagen and produce intermediate collagen products II and/or III.

Furthermore, intermediate collagen production methods and collagen implant production methods described above may include some or all of the steps in any order. For example, an intermediate collagen product may include the liquid component of the blended collagen dispersion containing a leveling agent and not the foam component. Such an intermediate product may be useful when preparing composite collagen products, for example, that have a collagen product made from only the collagen foam, and a collagen product made only from the collagen dispersion left after the foam is removed. Moreover, although the products described above have associated exemplary applications, other applications for the products are also contemplated. For example, wound repair matrices resembling a sponge or a film may serve as a growth media or substrate (e.g., stem cell growth media).

The above-described structural implants and method of making the implants that include human-derived or human-like collagen products should not be construed as limiting. For example, additional collagen types may be used in addition to or a an alternative to human-derived collagen. In some embodiments, collagen products may be prepared from genetically modified animals in a manner that renders the collagen products non-immunogenic, or that renders collagen- products having small amounts of antigenic components. In a particular example, collagen products derived from genetically modified pigs, which have no functional expression of the alpha 1,3 galactosyl transferase gene, may be used as a source of collagen. Furthermore, collagen products may be recovered from bovine, goat, sheep, or any animal genetically modified for use in humans. In another example, animal collagen products that have been enzymatically treated to remove glycoproteins to make the collagen substantially similar to human collagen may be used in accordance with some embodiments. In another example a substantially non-immunogenic collagen-containing soft tissue xenograft may be used as a starting material, and is disclosed in U.S. Patent No. 6,455,309, issued September 24, 2002, entitled "Proteoglycan-reduced soft tissue xenografts". Collagen may also be grown in cell cultures (e.g., recombinant collagen), which may be engineered to possess human or human-like characteristics. In a further example, xenograft placenta may comprise a source of collagen, which may be used as a collagen product implant alone or in combination with collagen derived from humans, e.g., human placenta. Collagen fibers and/or threads sourced from human collagen-containing tissue or human-like or from the above-described genetically modified or otherwise treated collagen used to form the products described are believed to be less likely to produce an immunogenic response when used for implantation into humans, and thus are likely to be accepted at an implant site.

The above-described structural implants should not be construed as limiting. For example, according to certain embodiments, various products having human-derived or human-like collagen product fibers and/or threads may be combined to form a composite of two or more of the above-mentioned products. In one example, a collagen product thread may be combined with a collagen product scaffold/matrix, each which may be produced using the same or a different intermediate collagen product as a starting material. In another example, collagen films may be combined with a collagen product scaffold/matrix by incorporating the film into and/or on the collagen product scaffold/matrix. In a further example, collagen product fibers, threads, fibrils and/or particles may be combined with each other, or may be combined with a collagen film, scaffold, etc. Other products not having human-derived or human-like collagen product fibers and/or threads may also be combined with the various products described herein. Moreover, although the products described above have associated exemplary applications, other applications for the products are also contemplated. For example, wound repair scaffolds resembling a sponge or a collagen product film may serve as a growth media or substrate. In addition, medical implants having human-derived or human-like collagen fibers and/or threads may be formed as a flexible or rigid implant depending on the implant's intended application.

Furthermore, products incorporating human-derived or human-like collagen fibers and/or threads may be designed to include various physical characteristics. For example, structural repair implants having incorporated collagen product fibers and/or threads may be constructed so that the implant is suturable, e.g., where the patch is produced to include suture holes in the non-woven fabric 701 seen in Figure 7, such that the implant can be fixed at an implant site. In addition, medical implants incorporating human-derived or human-like collagen product fibers and/or threads may be formed as a flexible or rigid implant depending on the implant's intended application. In another example, human-derived or human-like collagen products may be mixed with synthetic collagen or other synthetic biocompatible substances in order to achieve a desired product, physical property or performance. In a particular example, a synthetic, collagen product, or synthetic/collagen product fabric and/or scaffold may be incorporated with a collagen product scaffold, which may be implanted or compressed to yield a low profile material suitable for implantation. In another example, a collagen product is mixed with elastin from any source, or from humans, bovine, and/or porcine sources to yield products having particular strength characteristics. In addition, human-derived or human-like collagen products may be processed into putties or pastes so that the implant may be melted and/or shaped for an appropriate implantation use.

In accordance with some embodiments, other additives, including but not limited to those described below, may be added as a supplement to the human collagen products. It will be appreciated that the amount of additive used will vary depending upon the type of additive, the specific activity of the particular additive preparation employed, and the intended use of the composition. Any of a variety of medically and/or surgically useful optional substances can be added to, or associated with, the collagen product material, at any appropriate stage of the processing.

For example, angiogenesis may be an important contributing factor for the collagen product device in certain applications. In certain embodiments, angiogenesis is promoted so that blood vessels are formed at an implant site to allow efficient transport of oxygen and other nutrients and growth factors to the developing bone or cartilage tissue. Thus, angiogenesis promoting factors may be added to the collagen product to increase angiogenesis. For example, class 3 semaphorins, e.g., SEMA3, controls vascular morphogenesis by inhibiting integrin function in the vascular system, Serini et al., Nature, (July 2003) 424:391-397, and may be included in the collagen product device.

In accordance with other embodiments, collagen product devices may be supplemented, further treated, or chemically modified with one or more bioactive agents or bioactive compounds. Bioactive agent or bioactive compound, as used herein, refers to a compound or entity that alters, inhibits, activates, or otherwise affects biological or chemical events. For example, bioactive agents may include, but are not limited to, osteogenic or chondrogenic proteins or peptides; demineralized bone powder as described in U.S. Pat. No. 5,073,373; hydroxyapatite and/or other minerals; xenogenic collagen products, insoluble collagen product derivatives, etc., and soluble solids and/or liquids dissolved therein; anti-AIDS substances; anti-cancer substances; antimicrobials and/or antibiotics such as erythromycin, bacitracin, neomycin, penicillin, polymycin B, tetracyclines, biomycin, chloromycetin, and streptomycins, cefazolin, ampicillin, azactam, tobramycin, clindamycin and gentamycin, etc.; immunosuppressants; anti-viral substances such as substances effective against hepatitis; enzyme inhibitors; hormones; neurotoxins; opioids; hypnotics; anti-histamines; lubricants; tranquilizers; anti-convulsants; muscle relaxants and anti-Parkinson substances; anti-spasmodics and muscle contractants including channel blockers; miotics and anti-cholinergics; anti-glaucoma compounds; anti-parasite and/or anti-protozoal compounds; modulators of cell-extracellular matrix interactions including cell growth inhibitors and antiadhesion molecules; vasodilating agents; inhibitors of DNA, RNA, or protein synthesis; anti-hypertensives; analgesics; anti-pyretics; steroidal and non-steroidal anti-inflammatory agents; anti-angiogenic factors; angiogenic factors and polymeric carriers containing such factors; anti-secretory factors; anticoagulants and/or antithrombotic agents; local anesthetics; ophthalmics; prostaglandins; antidepressants; anti-psychotic substances; anti-emetics; imaging agents; biocidal/biostatic sugars such as dextran, glucose, etc.; amino acids; peptides; vitamins; inorganic elements; co-factors for protein synthesis; endocrine tissue or tissue fragments; synthesizers; enzymes such as alkaline phosphatase, collagenase, peptidases, oxidases, etc.; polymer cell scaffolds with parenchymal cells; collagen lattices; antigenic agents; cytoskeletal agents; cartilage fragments; living cells such as chondrocytes, bone marrow cells, mesenchymal stem cells; natural extracts; genetically engineered living cells or otherwise modified living cells; expanded or cultured cells; DNA delivered by plasmid, viral vectors, or other means; tissue transplants; autogenous tissues such as blood, serum, soft tissue, bone marrow, etc.; bioadhesives; BMPs; osteoinductive factor (IFO); fibronectin (FN); endothelial cell growth factor (ECGF); vascular endothelial growth factor (VEGF); cementum attachment extracts (CAE); ketanserin; human growth hormone (HGH); animal growth hormones; epidermal growth factor (EGF); interleukins, e.g., interleukin-1 (IL-1), interleukin-2 (IL-2); human alpha thrombin; transforming growth factor (TGF-beta); insulin-like growth factors (IGF-1, IGF-2); parathyroid hormone (PTH); platelet derived growth factors (PDGF); fibroblast growth factors (FGF, BFGF, etc.); periodontal ligament chemotactic factor (PDLGF); enamel matrix proteins; growth and differentiation factors (GDF); hedgehog family of proteins; protein receptor molecules; small peptides derived from growth factors above; bone promoters; cytokines; somatotropin; bone digesters; antitumor agents; cellular attractants and attachment agents; immuno-suppressants; permeation enhancers, e.g., fatty acid esters such as laureate, myristate and stearate monoesters of polyethylene glycol, enamine derivatives, alpha-keto aldehydes, etc.; and nucleic acids.

In certain embodiments, the bioactive agent may be a drug. In some embodiments, the bioactive agent may be a growth factor, cytokine, extracellular matrix molecule, or a fragment or derivative thereof, for example, a cell attachment sequence such as RGD. A more complete listing of bioactive agents and specific drugs suitable for use in the present invention may be found in "Pharmaceutical Substances: Syntheses, Patents, Applications" by Axel Kleemann and Jurgen Engel, Thieme Medical Publishing, 1999; the "Merck Index: An Encyclopedia of Chemicals, Drugs, and Biologicals", Edited by Susan Budavari et al., CRC Press, 1996; and the United States Pharmacopeia-25/National Formulary-20, published by the United States Pharmcopeial Convention, Inc., Rockville MD, 2001.

In some embodiments, the agent to be delivered may be adsorbed to or otherwise associated with the human collagen. The agent may be associated with the collagen product through specific or non-specific interactions, covalent or noncovalent interactions, etc. Examples of specific interactions include those between a ligand and a receptor, an epitope and an antibody, etc. Examples of non-specific interactions include hydrophobic interactions, electrostatic interactions, magnetic interactions, dipole interactions, van der Waals interactions, hydrogen bonding, etc. In certain embodiments, the agent may be attached to the collagen product using a linker so that the agent is free to associate with its receptor or site of action in vivo. In other embodiments, the agent may be bound or captured within the collagen product as a result of collagen cross-linking. In certain embodiments, the agent to be delivered may be attached to a chemical compound such as a peptide. In another embodiment, the agent to be delivered may be attached to an antibody, or fragment thereof, that recognizes an epitope found within the collagen. In certain embodiments, at least two bioactive agents may be attached to the collagen product. In other embodiments, at least three bioactive agents may be attached to the collagen product. Sebald et al., PCT/EP00/00637, describes the production of exemplary engineered growth factors that are beneficial for use with the collagen device.

While the present disclosure is written primarily in terms of human tissue and human collagen, it is understood that some methods may be used in any appropriate context with any appropriate material. The present invention is directed to any type of tissue that may be implanted in an allogenic context in any vertebrate species. For example, equine collagen may be processed and used for equine implantation, canine collagen may be processed and used for canine implantation, etc. The use of tissue for implantation from the same species source can provide benefits due to the potential of the natural constituents, unique to the species, providing implantation benefits once implanted. For example, a biochemical response in the implantee recognizing the natural constituents in the implant as acceptable may facilitate biological processes such as cross-linking and integration.

## Claims

1. A method for preparing a human derived collagen product, comprising:
treating harvested human tissue with ficin to form a collagen product;
deactivating the ficin with a non-alkaline enzyme deactivation solution; and
collecting the collagen product resulting from the ficin treatment, the collected collagen product having a preserved amount of its natural collagen constituents.

2. The method of claim 1, wherein the harvested tissue-containing collagen is selected from a tendon, pericardial tissue, intestinal tissue or fascia.

3. The method of claim 1, wherein the harvested tissue-containing collagen contains type I collagen or type III collagen or type V collagen or two or more of type I collagen, type III collagen, and type V collagen.

4. The method of claim 1, wherein the non-alkaline enzyme deactivation solution is selected from sodium chlorite in an ammonium nitrate buffer solution or hydrogen peroxide in a sodium chlorite buffer solution.

5. The method of claim 1, wherein the collagen product is processed to include a bioactive agent.

6. A method of forming a medical implant, comprising preparing a human derived oollagen product according to any one of claims 1 to 5 and forming the collagen product into the medical implant.

7. The method of claim 6, comprising:
causing an enzymatically-treated human derived collagen product having a preserved amount of its natural constituents in a solution to react to form a collagen thread, wherein the enzyme treatment comprises digestion of human collagen-containing tissue with ficin and deactivation of the ficin with a non-alkaline enzyme deactivation solution; and
forming a collagen fabric from the collagen thread.

8. The method of claim 1, 6 or 7, wherein the human-derived collagen product is formed of fibers and / or threads.

9. The method of claim 7, wherein said collagen fabric is a woven collagen fabric; wherein said collagen fabric is, alternatively, a non- woven collagen fabric.

10. The method of claim 6, comprising .preparing a human derived collagen product according to any one of claims 1 to 5; dispersing in solution the human derived collagen product; forming the dispersed collagen product into a medical implant; and removing the liquid component of the collagen product dispersion.

11. The method of claim 10, wherein dispersing human derived collagen product comprises at least suspending the human derived collagen product in a solution.

12. The method of claim 10, wherein said liquid component is removed by evaporating.

13. The method of claim 12, wherein evaporating the liquid component of the collagen product dispersion comprises lyophilizing the collagen product dispersion.

14. The method of claim 13, wherein evaporating the liquid component of the collagen product dispersion comprises freezing and lyophilizing the collagen product dispersion.

15. The method of claim 10, further comprising cross-linking the collagen dispersion.

## Patentansprüche

1. Verfahren zur Herstellung eines Kollagenprodukts menschlicher Abstammung, das Folgendes umfasst:
Behandeln von entnommenem menschlichem Gewebe mit Ficin, um ein Kollagenprodukt zu bilden
Deaktivieren des Ficins mit einer nichtalkalischen Enzym-Deaktivierungslösung; und
Sammeln des Kollagenprodukts, das durch die Behandlung mit Ficin entsteht, wobei das gesammelte Kollagenprodukt eine beibehaltene Menge seiner natürlichen Kollagenbestandteile aufweist.

2. Verfahren nach Anspruch 1, worin das entnommene Gewebe, das Kollagen enthält, aus einer Sehne, Perikardgewebe, intestinalem Gewebe oder Faszie ausgewählt ist.

3. Verfahren nach Anspruch 1, worin das entnommene Gewebe, das Kollagen enthält, Kollagen Typ I Kollagen oder Typ III Kollagen oder Typ V Kollagen oder zwei oder mehr von Typ I Kollagen, Typ III Kollagen und Typ V Kollagen enthält.

4. Verfahren nach Anspruch 1, worin die nichtalkalische Enzym-Deaktivierungslösung aus Natriumchlorit in einer Ammoniumnitrat-Pufferlösung oder Wasserstoffperoxid in einer Natriumchlorit-Pufferlösung ausgewählt ist.

5. Verfahren nach Anspruch 1, worin das Kollagenprodukt verarbeitet wird, um ein biologisch aktives Mittel einzuschließen.

6. Verfahren zur Bildung eines medizinischen Implantats, das die Herstellung eines Kollagenprodukts menschlicher Abstammung nach einem der Ansprüche 1 bis 5 und die Bildung des medizinischen Implantats aus dem Kollagenprodukt umfasst.

7. Verfahren nach Anspruch 6, das Folgendes umfasst:
Herbeiführen einer Reaktion eines enzymatisch behandelten Kollagenprodukts menschlicher Abstammung, das eine beibehaltene Menge seiner natürlichen Kollagenbestandteile aufweist, in einer Lösung, um einen Kollagen-Faden zu bilden, worin die Enzymbehandlung die Verdauung von menschlichem Kollagen-haltigem Gewebe mit Ficin und die Deaktivierung des Ficins mit einer nichtalkalischen Enzym-Deaktivierungslösung umfasst; und
Bilden eines Kollagen-Gewebes aus dem Kollagen-Faden.

8. Verfahren nach Anspruch 1, 6 oder 7, worin das Kollagenprodukt menschlicher Abstammung aus Fasern und/oder Fäden gebildet wird.

9. Verfahren nach Anspruch 7, worin das genannte Kollagen-Gewebe ein gewebtes Kollagen-Gewebe ist, worin das genannte Kollagen-Gewebe alternativ ein nicht gewebtes Kollagen-Gewebe ist.

10. Verfahren nach Anspruch 6, das Folgendes umfasst: Herstellen eines Kollagenprodukts menschlicher Abstammung nach einem der Ansprüche 1 bis 5; Dispergieren des Kollagenprodukts menschlicher Abstammung in Lösung; Bilden eines medizinischen Implantats aus dem dispergierten Kollagenprodukt; und Entfernen der flüssigen Komponente der Kollagenproduktdispersion.

11. Verfahren nach Anspruch 10, worin das Dispergieren des Kollagenprodukts menschlicher Abstammung zumindest das Suspendieren des Kollagenprodukts menschlicher Abstammung in einer Lösung umfasst.

12. Verfahren nach Anspruch 10, worin die genannte flüssige Komponente durch Verdampfen entfernt wird.

13. Verfahren nach Anspruch 12, worin das Verdampfen der flüssigen Komponente der Kollagenproduktdispersion das Lyophilisieren der Kollagenproduktdispersion umfasst.

14. Verfahren nach Anspruch 13, worin das Verdampfen der flüssigen Komponente der Kollagenproduktdispersion das Gefrieren und Lyophilisieren der Kollagenproduktdispersion umfasst.

15. Verfahren nach Anspruch 10, das weiter das Vernetzen der Kollagendispersion umfasst.

## Revendications

1. Méthode de préparation d'un produit de collagène dérivé de l'homme, comprenant :
le traitement par de la ficine de tissu humain prélevé, afin de former un produit de collagène ;
la désactivation de la ficine par une solution de désactivation enzymatique non alcaline ; et
la récupération du produit de collagène résultant du traitement par la ficine, le produit de collagène récupéré ayant une quantité conservée de ses constituants de collagène naturels.

2. Méthode selon la revendication 1, dans laquelle le tissu prélevé contenant du collagène est choisi parmi un tendon, un tissu péricardique, un tissu intestinal ou un fascia.

3. Méthode selon la revendication 1, dans laquelle le tissu prélevé contenant du collagène contient du collagène de type I ou du collagène de type III ou du collagène de type V ou deux, ou plus, parmi du collagène de type I, du collagène de type III et du collagène de type V.

4. Méthode selon la revendication 1, dans laquelle la solution de désactivation enzymatique non alcaline est choisie parmi le chlorite de sodium dans une solution tampon de nitrate d'ammonium ou du peroxyde d'hydrogène dans une solution tampon de chlorite de sodium.

5. Méthode selon la revendication 1, dans laquelle le produit de collagène est traité pour inclure un agent bioactif.

6. Méthode de formation d'un implant médical, comprenant la préparation d'un produit de collagène dérivé de l'homme selon l'une quelconque des revendications 1 à 5, et la mise en forme du produit de collagène en implant médical.

7. Méthode selon la revendication 6, comprenant :
le fait de provoquer la réaction en solution d'un produit de collagène dérivé de l'homme et traité enzymatiquement, ayant une quantité conservée de ses constituants naturels, afin de former un fil de collagène, où le traitement enzymatique comprend la digestion de tissu contenant du collagène humain par de la ficine, et la désactivation de la ficine par une solution de désactivation enzymatique non alcaline ; et
la formation d'un tissu de collagène à partir du fil de collagène.

8. Méthode selon la revendication 1, 6 ou 7, dans laquelle le produit de collagène dérivé de l'homme est formé de fibres et/ou de fils.

9. Méthode selon la revendication 7, dans laquelle ledit tissu de collagène est un tissu de collagène tissé, où ledit tissu de collagène est, de manière alternative, un tissu de collagène non tissé.

10. Méthode selon la revendication 6, comprenant la préparation d'un produit de collagène dérivé de l'homme selon l'une quelconque des revendications 1 à 5 ; la dispersion en solution du produit de collagène dérivé de l'homme ; la mise en forme du produit de collagène dispersé en implant médical ; et l'élimination du composant liquide de la dispersion du produit de collagène.

11. Méthode selon la revendication 10, dans laquelle la dispersion du produit de collagène dérivé de l'homme comprend au moins la suspension du produit de collagène dérivé de l'homme dans une solution.

12. Méthode selon la revendication 10, dans laquelle ledit composant liquide est éliminé par évaporation.

13. Méthode selon la revendication 12, dans laquelle l'évaporation du composant liquide à partir de la dispersion du produit de collagène comprend la lyophilisation de la dispersion du produit de collagène.

14. Méthode selon la revendication 13, dans laquelle l'évaporation du composant liquide à partir de la dispersion du produit de collagène comprend la congélation et la lyophilisation de la dispersion du produit de collagène.

15. Méthode selon la revendication 10, comprenant en outre la réticulation de la dispersion de collagène.
